(19) Europäisches Patentamt
European Patent Office
Office européen des brevets

(11) **EP 2 954 925 A1**

(12) **EUROPÄISCHE PATENTANMELDUNG**

(43) Veröffentlichungstag:
**16.12.2015   Patentblatt 2015/51**

(51) Int Cl.:
***A61M 37/00*** *(2006.01)*

(21) Anmeldenummer: **14172119.1**

(22) Anmeldetag: **12.06.2014**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(71) Anmelder: **MT.DERM GmbH**
**14167 Berlin (DE)**

(72) Erfinder: **Scherkowski, Dirk**
**12437 Berlin (DE)**

(74) Vertreter: **Bittner, Thomas L.**
**Boehmert & Boehmert**
**Anwaltspartnerschaft mbB**
**Patentanwälte Rechtsanwälte**
**Pettenkoferstrasse 20-22**
**80336 München (DE)**

Bemerkungen:
Geänderte Patentansprüche gemäss Regel 137(2) EPÜ.

(54) **Handgerät zum wiederholten Aufstechen einer menschlichen oder tierischen Haut und Antriebsmodul**

(57)   Die Anmeldung betrifft ein Handgerät zum wiederholten lokalen Aufstechen einer menschlichen oder tierischen Haut, mit einem Gehäuse (2), einer Antriebseinrichtung, einem Wandlungsmechanismus, und einer Stecheinrichtung, die eine oder mehrere Stechnadeln aufweist, wobei der Wandlungsmechanismus eine Kurbeleinrichtung (20) aufweist, die an die Antriebswelle koppelt, und eine Reihenanordnung (21) von mehreren gelenkig miteinander gekoppelten Pleuelelementen aufweist, wobei bei der Reihenanordnung (21) ein proximales Pleuelelement (22) mit der Kurbeleinrichtung (20) und ein distales Pleuelelement (23) mit dem Nadelschaft in Verbindung steht, derart, dass im Betrieb die axiale ausgerichtete Antriebsbewegung auf den Nadelschaft eingeleitet wird.

Fig. 3

EP 2 954 925 A1

**Beschreibung**

Die Erfindung betrifft ein Handgerät zum wiederholten Aufstechen einer menschlichen oder tierischen Haut sowie ein Antriebsmodul für ein solches Handgerät.

Hintergrund

Vorrichtungen zum lokalen Aufstechen einer menschlichen oder einer tierischen Haut werden in der Regel als Handgeräte ausgeführt. Vom Bedienpersonal können derartige Handgeräte zum Aufbringen einer Farbe für eine Tätowierung (Tattoo) und / oder permanentes Make-up im Bereich der Hautoberfläche verwendet werden. Aber auch das Einbringen kosmetischer oder medizinischer Wirkstoffe über die Haut ist mit solchen Geräten möglich, indem die Haut lokal aufgestochen wird. Darüber hinaus können solche Geräte verwendet werden, ohne dass irgendeine Substanz eingebracht wird, zum Beispiel zur Hautstimulierung.

Ein Handgerät zum lokalen Aufstechen einer Haut ist beispielsweise aus der Druckschrift DE 299 19 199 U1 bekannt. Das bekannte Handgerät weist ein Griffstück, eine Antriebseinrichtung und eine Stechnadel auf, die mit Hilfe der Antriebseinrichtung im Betrieb relativ zu einer Nadeldüse vor und zurück bewegt wird, wobei mindestens zwei lösbar miteinander verbundene Module vorgesehen sind, und das eine der beiden Module als wieder verwendbares Grundmodul mit integrierter Antriebseinrichtung ausgebildet ist. Das andere der beiden Module ist ein sterilisiertes Einwegmodul, in das bei dem bekannten Handgerät alle durch die Köperflüssigkeiten eines Kunden infizierbaren Bestandteile integriert sind. Auf diese Weise wird das Handgerät in Form von zwei Modulen zur Verfügung gestellt, von denen eines, nämlich das Einwegmodul, nach der Benutzung ausgetauscht werden kann, während das andere Modul, welches die Antriebseinrichtung umfasst, wieder verwendet wird. Mit Hilfe des Einwegmoduls werden die hygienischen Bedingungen beim Aufbringen einer Tätowierung und / oder von permanentem Make-up verbessert, da alle Teile ausgetauscht werden, die potentiell durch die bei der Behandlung austretende Körperflüssigkeit des Kunden kontaminiert werden können. Es wird so vermieden, dass das gesamte Handgerät ausgetauscht werden muss.

Aus dem Dokument EP 1 495 782 A1 ist ein Antriebsmodul für eine Vorrichtung zum lokalen Aufstechen einer menschlichen oder einer tierischen Haut bekannt, bei dem eine Antriebseinrichtung, mit der eine Antriebsbewegung erzeugbar ist, und ein an die Antriebseinrichtung gekoppelter Wandlungsmechanismus vorgesehen sind, mit dem die Antriebsdrehbewegung in einer auf eine die Haut lokal aufstechende Stecheinrichtung einkoppelbare Vorwärts- / Rückwärtsbewegung umgewandelt wird, so dass eine repetierende Bewegung einer Stechnadel ermöglicht ist. Der Wandlungsmechanismus umfasst ein Funktionsbauteil, welches bei der Bewegungswandlung eine Taumel- oder Kippbewegung ausführt, wodurch eine Antriebskraft zum Bewegen einer die Haut lokal aufstechenden Nadel in Vorwärts- und Rückwärtsrichtung zur Verfügung gestellt wird. Das Funktionsbauteil ist in einer Ausführung mittels eines Kugellagers freilaufend gelagert. Ein nicht beabsichtigtes Verdrehen des Funktionsbauteils, welches durch die Antriebsdrehbewegung hervorgerufen werden kann, wird bei der bekannten Vorrichtung dadurch verhindert, dass das mittels Kugellager montierte Funktionsbauteil oder ein hieran gebildeter Vorsprung in eine Ausnehmung eingreifen. Es hat sich gezeigt, dass diese Art der Sicherung des Funktionsbauteils beim Betrieb der Vorrichtung zu einer nicht unerheblichen Geräuschbelastung führt, die insbesondere durch das Hin- und Herbewegen des Vorsprungs in der Ausnehmung verursacht wird.

Zusammenfassung

Aufgabe der Erfindung ist es, ein Handgerät zum wiederholten lokalen Aufstechen einer menschlichen oder tierischen Haut sowie ein Antriebsmodul hierfür anzugeben, bei denen im Betrieb des Handgerätes der Bedienkomfort verbessert ist, insbesondere hinsichtlich störender Begleiterscheinungen wie zum Beispiel Geräusche oder Vibrationen des Handgerätes.

Zur Lösung der Aufgabe ist ein Handgerät zum wiederholten lokalen Aufstechen einer menschlichen oder tierischen Haut nach dem unabhängigen Anspruch 1 geschaffen. Weiterhin betrifft der unabhängige Anspruch 15 ein Antriebsmodul für ein solches Handgerät. Vorteilhafte Ausgestaltungen sind Gegenstand von abhängigen Unteransprüchen.

Es ist ein Handgerät zum wiederholten lokalen Aufstechen einer menschlichen oder tierischen Haut geschaffen, welches ein Gehäuse mit einem hieran gebildeten Handgriff aufweist. Im Betrieb des Handgerätes, welches im Fall der Nutzung zum Ausbilden von Tattoos oder permanenten Makeup auch als Tätowiervorrichtung bezeichnet werden kann, greift der Nutzer den Handgriff, um das Handgerät dann zu führen. Andere Anwendungen sehen das lokale Aufstechen der Haut zum Einbringen eines kosmetischen oder medizinischen Wirkstoffes vor. Aber auch für das lokale Aufstechen der menschlichen oder tierischen Haut ohne Einbringen irgendeiner Substanz kann das Handgerät genutzt werden, zum Beispiel zur Hautstimulierung. Im Gehäuse ist eine Antriebseinrichtung aufgenommen, mit der über eine Antriebswelle eine rotierende Antriebskraft bereitgestellt wird. Hierfür kann ein Elektromotor vorgesehen sein.

An die Antriebswelle koppelt ein Wandlungsmechanismus, der im Gehäuse angeordnet und eingerichtet ist, die rotierende Antriebsbewegung (rotierende Antriebskraft) in eine axial ausgerichtete Antriebsbewegung zu wandeln. Die hierdurch bereitgestellte Antriebskraft ist axial ausgerichtet.

Es ist weiterhin eine Stecheinrichtung vorgesehen, die im Gehäuse aufgenommen ist und eine oder mehrere Stechnadeln aufweist, die an einer Nadelaufnahme angeordnet sind. Im Fall von mehreren Stechnadeln können diese in Gruppen angeordnet sein, in denen die Nadeln einander nahestehen. Aber auch die Verteilung von Nadeln oder Nadelgruppen über die Fläche einer Nadelplatte kann vorgesehen sein. In diesem Fall ist die Nadelaufnahme mit einer Nadelplatte gebildet. Die Nadelaufnahme ist verbunden mit dem Wandlungsmechanismus und wird zusammen mit der einen oder den mehreren Stechnadeln im Betrieb entlang einer Bewegungsbahn wiederholt vor und zurück bewegt in axialer Richtung.

Der Wandlungsmechanismus weist eine Kurbeleinrichtung auf, die an die Antriebswelle koppelt. Auf diese Weise kann ein Kurbeltrieb realisiert werden. Der Wandlungsmechanismus verfügt weiterhin über mehrere gelenkig miteinander gekoppelte Pleuelelemente, die in einer Reihenanordnung hintereinander angeordnet sind. Bei der Reihenanordnung koppelt ein proximales Pleuelelement an die Kurbeleinrichtung. Ein distales Pleuelelement steht mit der Nadelaufnahme in Verbindung, derart, dass im Betrieb die axial ausgerichtete Antriebskraft auf die Nadelaufnahme eingeleitet wird. Die Verbindung zwischen dem distalen Pleuelelement und der Nadelaufnahme kann eine direkte Kopplung oder eine über ein oder mehrere zwischenliegende Koppelelemente vermittelte, indirekte Verbindung sein. Beispielsweise kann die Nadelaufnahme an ein in einer axialen Führung aufgenommenes Kolbenelement direkt oder vermittelt koppeln, welches seinerseits gelenkig mit dem distalen Pleuelelement verbunden ist.

Bei dem Handgerät kann die Antriebseinrichtung, insbesondere ein Elektromotor, in einem zum Handgriff am Gehäuse querstehenden Gehäuseabschnitt aufgenommen sein. Die Antriebseinrichtung kann eingerichtet sein, im Betrieb eine drehende Antriebsbewegung von etwa 1.800 min$^{-1}$ bis etwa 9.600 min$^{-1}$ (30 Hz bis 160 Hz) bereitzustellen.

Das Gehäuse kann aus einem einzigen Material oder einer Kombination verschiedener Materialien bestehen, wozu insbesondere Metall und Kunststoff gehören. Gehäuseabschnitte können lösbar montiert sein, um zum Beispiel Bereiche des Gehäuseinneren zu Austausch- oder Wartungszwecken freizugeben. Aus dem Gehäuse kann eine Kabelverbindung herausgeführt sein, die zum Anschluss des Handgerätes an ein externes Steuergerät dient. Die Kabelverbindung kann dem Anschließen eines Elektromotors an eine Energiequelle dienen, insbesondere über das Steuergerät. Die Kabelverbindung kann Datenkabel aufweisen, über die zwischen dem Handgerät und dem Steuergerät elektronische Daten austauschbar sind. Ein solcher Datenaustausch zwischen Handgerät und Steuergerät kann alternativ oder ergänzend auch über eine drahtlose Datenverbindung ausgeführt werden, zum Beispiel unter Verwendung der Bluetooth-Technologie. Steuergeräte für Handgeräte zum wiederholten lokalen Aufstechen einer menschlichen oder tierischen Haut sind als solche in verschiedenen Ausführungsformen bekannt und werden hier daher nicht näher erläutert.

Weiterhin ist ein Antriebsmodul für ein Handgerät zum wiederholten lokalen Aufstechen einer menschlichen oder tierischen Haut mit einem Modulgehäuse geschaffen, an dem ein Handgriff gebildet ist. In dem Modulgehäuse ist eine Antriebseinrichtung aufgenommen, mit der über eine Antriebswelle eine rotierende Antriebsbewegung bereitgestellt wird. An die Antriebswelle koppelt ein Wandlungsmechanismus, der zumindest teilweise in dem Modulgehäuse angeordnet und eingerichtet ist, die rotierende Antriebskraft in eine axial ausgerichtete Antriebskraft zu wandeln. An und / oder in dem Modulgehäuse ist eine Kopplungseinrichtung gebildet, die eingerichtet ist, an ein Stechmodul lösbar zu koppeln, sei es zum Beispiel mittels einer Clip-Verbindung oder einer Schraubverbindung. Ergänzend oder alternativ können Rastelemente vorgesehen sein.

In den verschiedenen Ausführungsformen kann die Kopplung zwischen Wandlungsmechanismus und Nadelaufnahme, die mit einem Nadelschaft gebildet sein kann, derart ausgeführt sein, dass im Betrieb die Nadelaufnahme aufgrund der axial ausgerichteten Antriebskraft nicht nur vorgefahren wird, sondern auch mittels des das Kopplungsbauteil umfassenden Wandlungsmechanismus zurückgezogen wird. Bei dieser Ausführungsform stellt der Wandlungsmechanismus selbst eine Rückstellkraft bereit. Alternativ oder ergänzend kann vorgesehen sein, dass die Rückstellkraft wenigstens teilweise von einem elastischen Element bereitgestellt wird, beispielsweise einer Feder oder einer Membran. Bei dieser Ausführung erfolgt das Aus- oder Vorfahren der Nadelaufnahme mit der einen oder den mehreren Stechnadeln gegen eine elastische Vorspannung, die ihrerseits dann zum Zurückbewegen beiträgt oder dieses allein bewirkt. Das elastische Element zieht sich nach dem Strecken beim Ausfahren der Stechnadeln selbsttätig wieder zusammen und bewirkt so die Rückbewegung der einen oder der mehreren Nadeln.

Der Wandlungsmechanismus kann eine Gehäuselagerung aufweisen, mit der das proximale Pleuelelement am Gehäuse gelagert ist. Zum Beispiel kann das proximale Pleuelelement an einem von einer Innenwand zum Gehäuseinnenraum hin vorstehenden Gehäuseabschnitt gelagert sein. Ergänzend zu der Gehäuselagerung kann eine weitere Gehäuselagerung für ein anderes der Pleuelelemente in der Reihenanordnung vorgesehen sein, zum Beispiel das proximale Pleuelelement. Auch hier kann dann eine lagernde Aufnahme des proximalen Pleuelelementes auf einer Innenseite des Gehäuses vorgesehen sein.

Bei einer Ausgestaltung kann vorgesehen sein, dass das proximale Pleuelelement an der Gehäuselagerung um eine Schwenkachse schwenkbar gelagert ist.

Das proximale Pleuelelement kann mittels der Gehäuselagerung entlang einer Führung, die am Gehäuse oder an dem proximalen Pleuelelement gebildet ist, verschiebbar gelagert sein. Mit der Führung kann eine gerade Verschiebebahn bereitgestellt sein. Entlang dieser kann im Betrieb dann das proximale Pleuelelement verlagert werden. Die Führung kann mit einer Ausnehmung oder einem Durchbruch am Gehäuse oder an dem proximalen Pleuelelement gebildet sein. Die Ausführungen hinsichtlich möglicher Ausgestaltungen der Gehäuselagerung gelten entsprechend für eine wahlweise vorgesehene weitere Gehäuselagerung. Mit Hilfe der verschiebbaren Gehäuselagerung kann wenigstens eine Verschiebe- oder Gleitlagerung für die Verbindungen der Pleuelelemente der Reihenanordnung bereitgestellt sein, sei es für wenigstens eine Verbindung zwischen benachbarten Pleuelelementen oder wenigstens eine der endseitigen Verbindungen der Reihenanordnung, über welche diese an andere Funktionselemente koppelt. Eine Verschiebe- oder Gleitlagerung kann aber auch getrennt von den jeweiligen endseitigen Verbindungen des Pleuelelementes vorgesehen sein. Zum Beispiel kann sich an dem Pleuelelement in einem Teilbereich zwischen den äußeren Enden eine in Längsrichtung des Pleuelelementes ausgebildete Führung in Form eines Durchbruches oder einer Ausnehmung erstrecken, in welche dann ein zugeordnetes Lagerelement eingreift, das am Gehäuse gebildet ist. Eine solche Verschiebe- oder Gleitlagerung kann zum Beispiel an dem proximalen Pleuelelement vorgesehen sein. Die Führung kann mittels einer äußeren und zumindest teilweise das Pleuelelement umgreifenden Gleitlagerung erfolgen. Hierzu kann seitlich vom Pleuelelement eine Führungsschiene auskragen und in einem dreh- / schwenkbaren Axialgleitlager des Lagerelements geführt laufen.

Die Gehäuselagerung kann auf einer von der Nadelaufnahme abgewandten Seite der Kurbeleinrichtung am Gehäuse gebildet sein. In Relation zu einer gedachten Verbindungslinie zwischen Kurbeleinrichtung und Nadelaufnahme kann die Gehäuselagerung bei dieser Ausführung auf einer Rückseite der Kurbeleinrichtung angeordnet sein.

Eine Ausgestaltung kann vorsehen, dass ein Koppelelement der Kurbeleinrichtung in einer Pleuelelementführung an dem proximalen Pleuelelement verschiebbar lagert. Das Koppelelement der Kurbeleinrichtung kann in der Pleuelelementführung in Längsrichtung des Pleuelelementes verschiebbar gelagert sein. Es kann sich dann um eine Verschiebe- oder Gleitlagerung handeln. Auch eine drehbare Lagerung des Koppelelements in der Kurbeleinrichtung ist denkbar, wodurch sich eine Rolllagerung in der Pleuelelementführung ergibt. Alternativ kann das proximale Pleuelelement an der Kurbeleinrichtung um eine Schwenkachse schwenkbar aufgenommen sein.

Eine Weiterbildung kann vorsehen, dass zumindest ein Pleuelelement der Reihenanordnung einen axialen Längenausgleich zulassend ausgeführt ist. Ein axialer Längenausgleich des Pleuelelementes kann auf verschiedene Art und Weise realisiert werden. Beispielsweise können Pleuelelementteile in axialer Richtung relativ zueinander verschiebbar angeordnet sein. Die Pleuelelementabschnitte können hierbei ineinandergreifend angeordnet sein. Es kann ein elastisches Element vorgesehen sein, zum Beispiel eine Feder, mit dem die Pleuelelementabschnitte gegen eine Streckung des Pleuelelementes vorgespannt sind, so dass von dem elastischen Element eine Rückstellkraft bereitgestellt werden kann, um das gestreckte Pleuelelement wieder zusammen zu ziehen.

Die Reihenanordnung kann aus dem proximalen Pleuelelement und dem distalen Pleuelelement bestehen. Bei dieser Ausführungsform beschränkt sich die Reihenanordnung auf zwei Pleuelelemente, die miteinander gekoppelt sind, wobei wenigstens ein Verschiebe- oder Gleitlager vorgesehen sein kann.

Bei einer Ausgestaltung kann vorgesehen sein, dass eine Hubeinstellvorrichtung vorgesehen ist, mit der ein Hub der axial ausgerichteten Antriebsbewegung einstellbar ist. Der Hub ist die axial ausgerichtete Bewegung zwischen den beiden Umkehrpunkten der Vor- und Zurückbewegung der Nadelaufnahme. Er beeinflusst, wie weit die Nadelspitze der einen oder der mehreren Stechnadeln vorwärts bewegt wird, wodurch in einer Ausgestaltung die Einstechtiefe im lokalen Bereich der aufzustechenden Haut beeinflusst werden kann. Mit Hilfe der Verstellbarkeit des Hubes der axial ausgerichteten Antriebsbewegung kann das Handgerät für unterschiedliche Anwendungszwecke eingestellt werden.

Mit Hilfe der Hubeinstellvorrichtung kann die Gehäuselagerung verstellbar sein. Hierbei kann vorgesehen sein, dass mit Hilfe der Hubeinstellvorrichtung eine Relativverlagerung zwischen Gehäuselagerung und der Achse der Antriebswelle erfolgt. Auf diese Weise verändert sich dann der am proximalen Pleuelelement zur Einkopplung auf den Nadelschaft bereitgestellte Hub. Zusätzlich kann darüber der funktionelle Zusammenhang zwischen der Position der Stecheinrichtung und der an dieser Position vorliegenden Geschwindigkeit derselben beeinflusst werden. Zum Verstellen der Hubeinstellvorrichtung kann ein Dreh- oder Schraubmechanismus vorgesehen sein.

Es kann vorgesehen sein, dass die Hubeinstellvorrichtung eine am Gehäuse außenliegend angeordnete Einstelleinrichtung aufweist.

Eine Ausgestaltung kann vorsehen, dass axial ausgerichtete Antriebsbewegung quer zur axialen Richtung der Antriebswelle verläuft.

Das Gehäuse kann mit mehreren Gehäusemodulen gebildet sein, wobei in einem Antriebsmodul die Antriebseinrichtung und in einem Stechmodul die Stecheinrichtung angeordnet ist. Die Gehäusemodule können lösbar miteinander verbunden sein. In einer Ausgestaltung von lösbar miteinander verbundenen Gehäusemodulen kann das Stechmodul, welches auch als Nadelmodul bezeichnet werden kann, als sterilisiertes Einwegmodul ausgeführt sein.

Bei einer Ausgestaltung kann vorgesehen sein, dass der Wandlungsmechanismus teilweise in dem Antriebsmodul und teilweise in dem Stechmodul gebildet ist. Hierbei kann vorgesehen sein, dass wenigstens das proximale Pleuelmodul in dem Antriebsmodul angeordnet ist, wohingegen wenigstens das distale Pleuelelement in dem Stechmodul aufgenommen ist. Sind Antriebsmodul und Stechmodul lösbar miteinander verbunden, kann zum Ausbilden der Reihenanordnung der Pleuelelemente beim Anbringen des Stechmoduls an dem Antriebsmodul zum Beispiel eine Steckverbindung zwischen den zu koppelnden Pleuelelementen vorgesehen sein.

In Verbindung mit dem Antriebsmodul für ein Handgerät zum wiederholten lokalen Aufstechen einer menschlichen oder tierischen Haut gelten die vorangehend im Zusammenhang mit Ausführungen des Handgerätes gemachten Erläuterungen entsprechend.

Beschreibung von Ausführungsbeispielen

Im Folgenden werden weitere Ausführungsbeispiele unter Bezugnahme auf Figuren einer Zeichnung näher erläutert. Hierbei zeigen:

| | |
|---|---|
| Fig. 1 | eine schematische Darstellung eines Handgeräts zum wiederholten lokalen Aufstechen einer menschlichen oder tierischen Haut von der Seite, |
| Fig. 2 | eine schematische Darstellung des Handgeräts aus Fig. 1 von oben, |
| Fig. 3 | eine perspektivische Darstellung eines Abschnitts des Handgeräts aus Fig. 1 im Aufriss, wobei Pleuelelemente in Längsrichtung ausgerichtet sind, |
| Fig. 4 | eine perspektivische Darstellung eines Abschnitts des Handgeräts aus Fig. 1 im Aufriss, wobei die Pleuelelemente schräg zueinander gestellt sind, |
| Fig. 5 bis 13 | Darstellungen des Handgeräts aus Fig. 1 im Aufriss von oben mit einer Pleuelstellung, die sich hinsichtlich eines zunehmenden Drehwinkels im Uhrzeigersinn unterscheiden, beginnend bei einer Stellung gemäß Fig. 3, |
| Fig. 14a bis d | schematische Darstellungen für Ausführungsformen eines Wandlungsmechanismus, welcher an eine Kurbeleinrichtung koppelt, |
| Fig. 14e | schematische Darstellungen einer weiteren Ausführungsform eines Wandlungsmechanismus, welcher an eine Kurbeleinrichtung koppelt, |
| Fig. 15 | eine schematische Darstellung einer geänderten Variante nach dem Beispiel 14.5, |
| Fig. 16 | eine schematische Darstellung zur Erläuterung einer Hubeinstellung, |
| Fig. 17 | eine schematische Darstellung zur Erläuterung der Hubeinstellung und |
| Fig. 18 | eine weitere schematische Darstellung zur Erläuterung der Variationen des Wandlungsmechanismus gemäß Fig. 14a. |

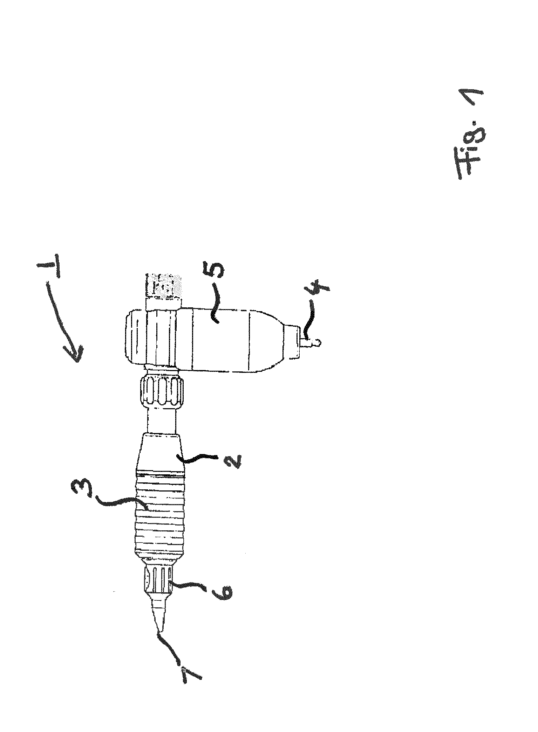

Fig. 1 zeigt eine schematische Darstellung eines Handgerätes 1 zum wiederholten lokalen Aufstechen einer menschlichen oder tierischen Haut. An einem Gehäuse 2 ist außen ein Handgriff 3 gebildet. Bei der dargestellten Ausführungsform ist in dem Gehäuse 2 ein Elektromotor als Antrieb aufgenommen, welcher über eine Steckereinrichtung 4 an eine Energieversorgung angeschlossen werden kann. Es kann vorgesehen sein, dass über die Steckereinrichtung 4 auch Signal- oder Steueranschlüsse realisiert werden, zum Beispiel zum Verbinden des Handgerätes mit einer externen Steuereinrichtung (nicht dargestellt), über die zum Beispiel eine Drehzahlregelung für den Elektromotor bereitgestellt sein kann.

Das Gehäuse 2 umfasst ein Antriebsmodul 5 sowie ein Stech- oder Nadelmodul 6, die lösbar miteinander verbunden sind, derart, dass das Stechmodul 6 ausgetauscht werden kann. Im Stechmodul 6 sind in üblicher Weise ein oder mehrere Nadeln aufgenommen, die im Betrieb durch eine vordere Gehäuseöffnung 7 aus- und eingefahren werden können. Die eine oder die mehreren Nadeln ihrerseits sind an einer Nadelaufnahme (nicht dargestellt) in üblicher Weise angeordnet.

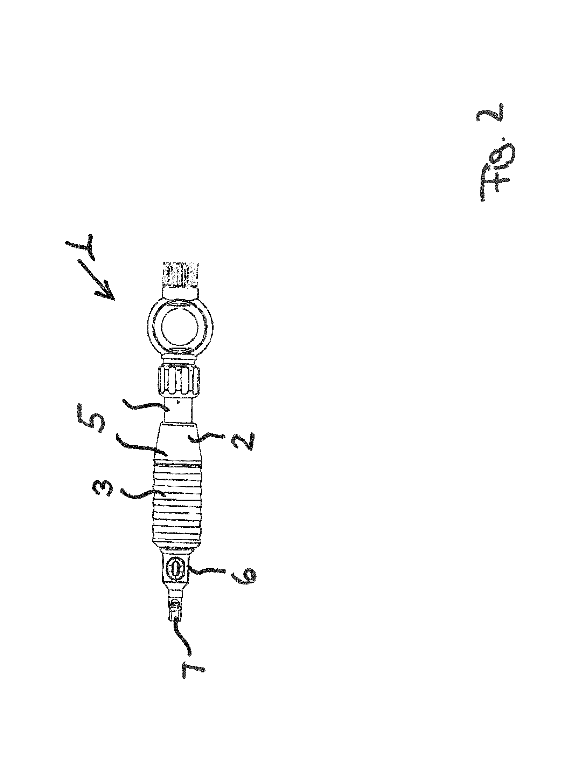

Fig. 2 zeigt das Handgerät aus Fig. 1 von oben.

Die Fig. 3 und 4 zeigen perspektivische Darstellungen eines Abschnitts des Handgeräts 1 aus Fig. 1 im Aufriss. Im Gehäuse 2 sind Funktionselemente einer Antriebseinrichtung aufgenommen, die insbesondere einen Elektromotor umfassen, über dessen Antriebswelle eine rotierende Antriebskraft bereitgestellt wird. Bei der gezeigten Ausführungsform ist ein Elektromotor in einem querstehenden Gehäuseteil 8 aufgenommen.

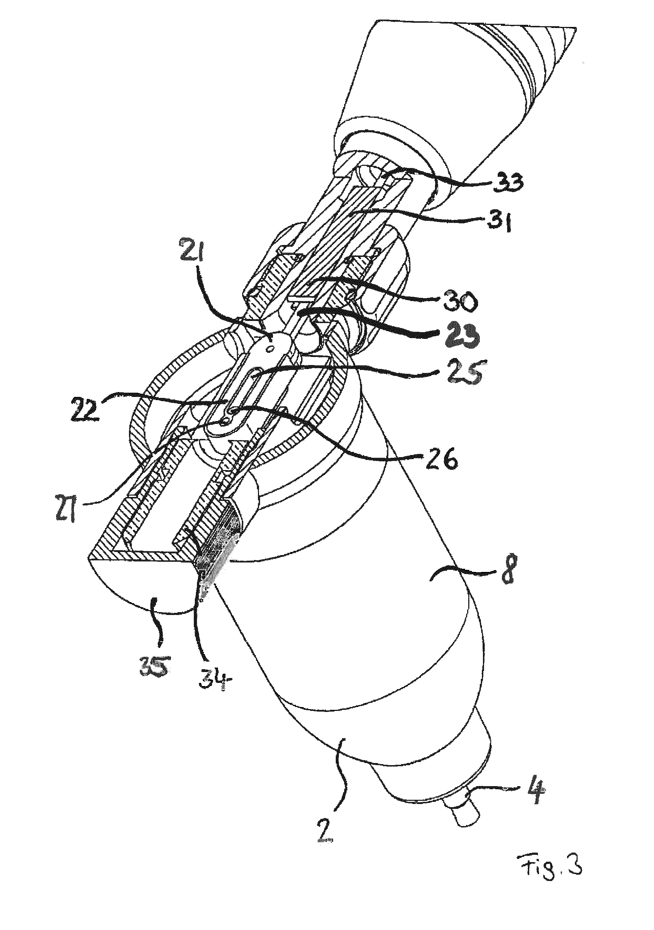

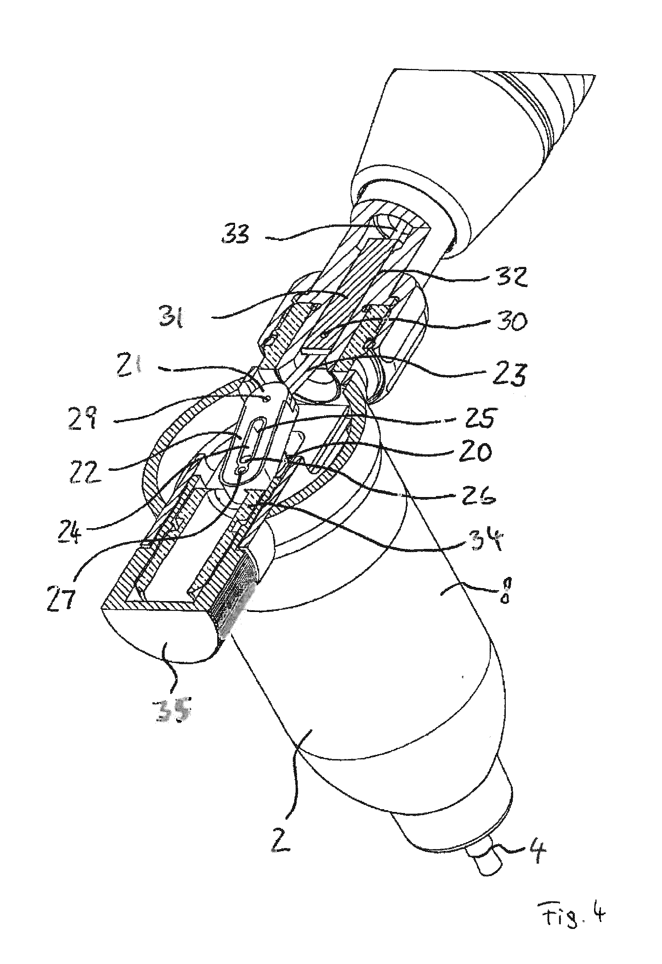

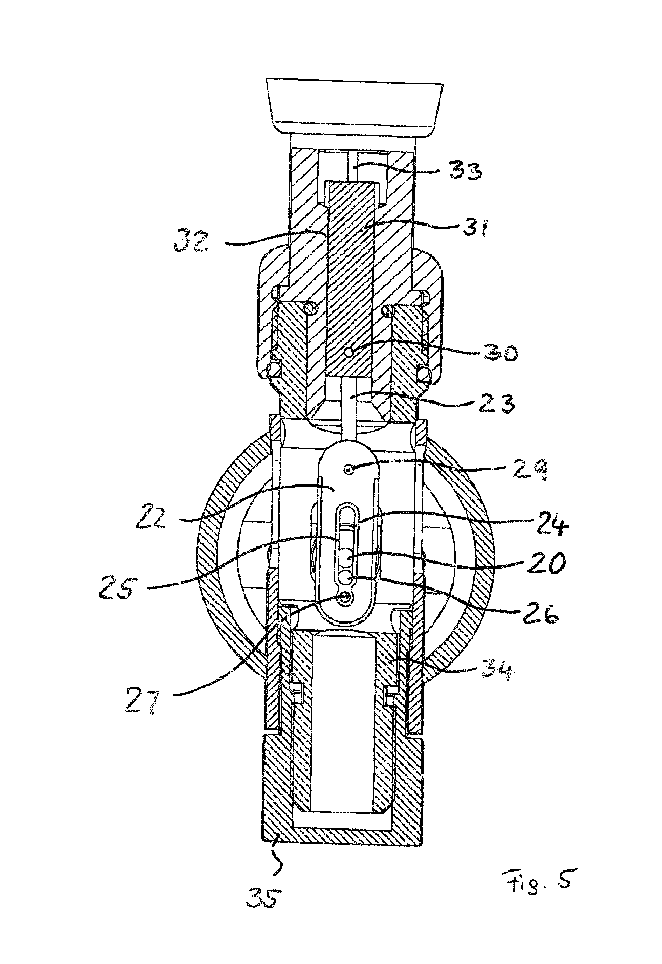

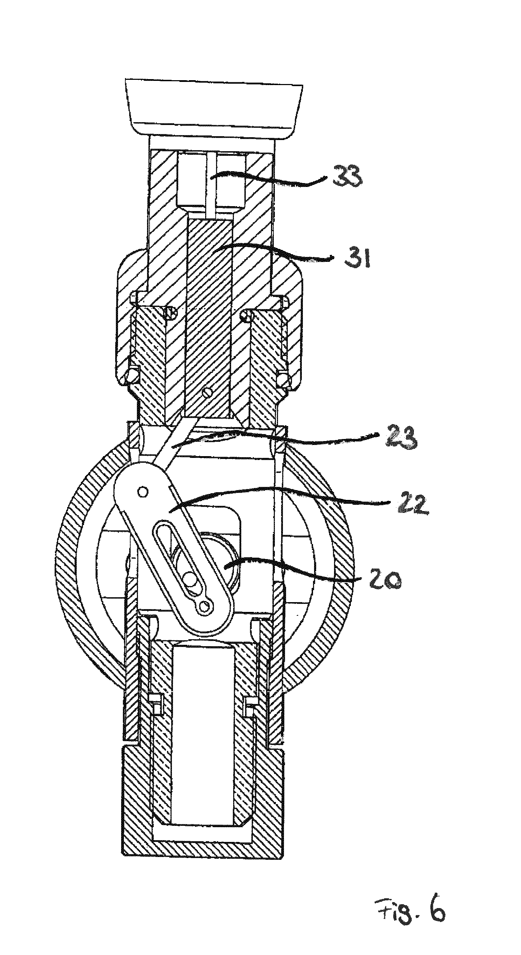

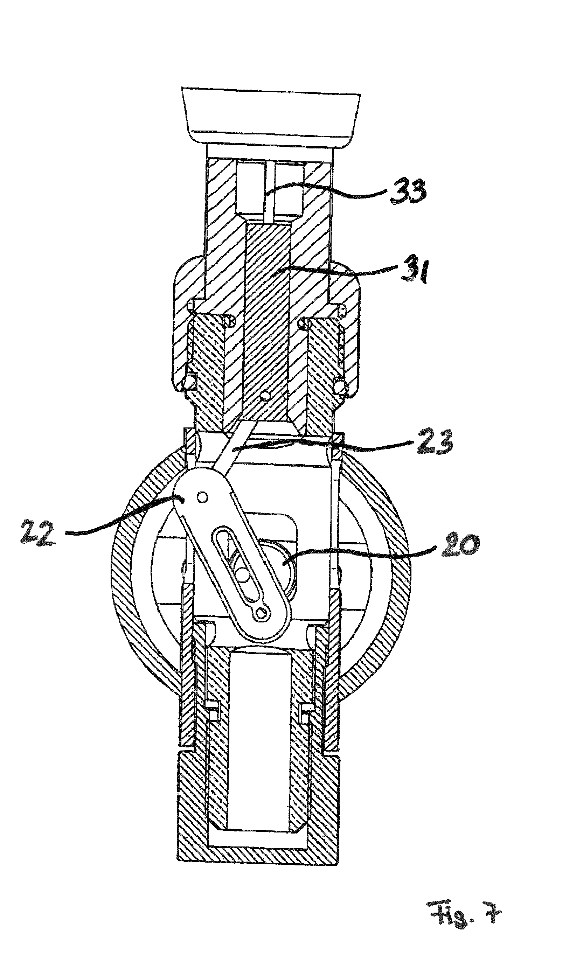

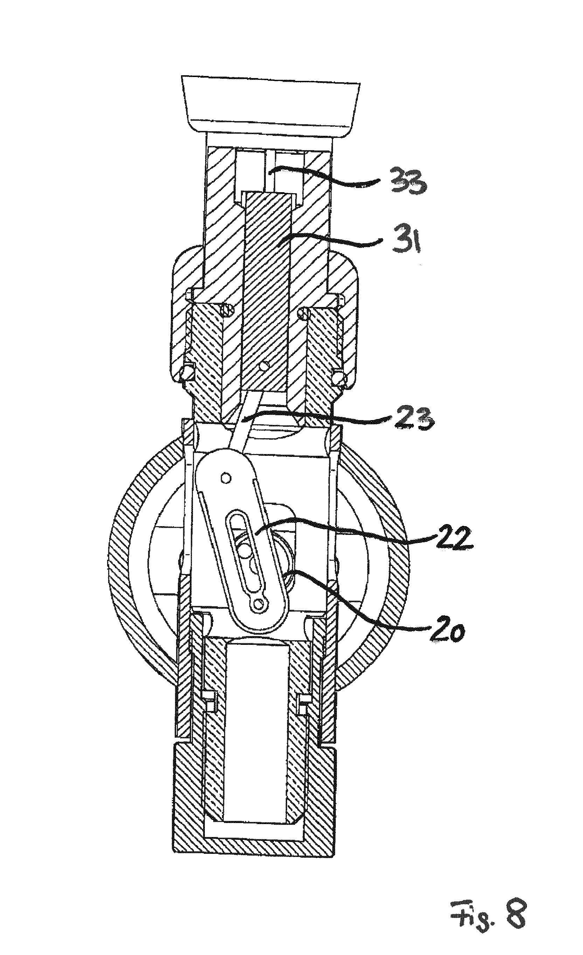

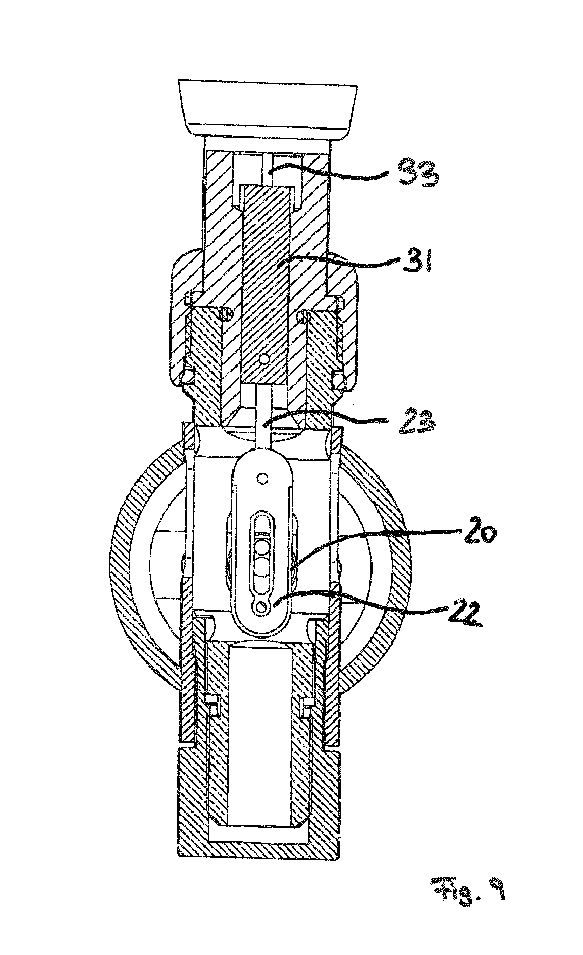

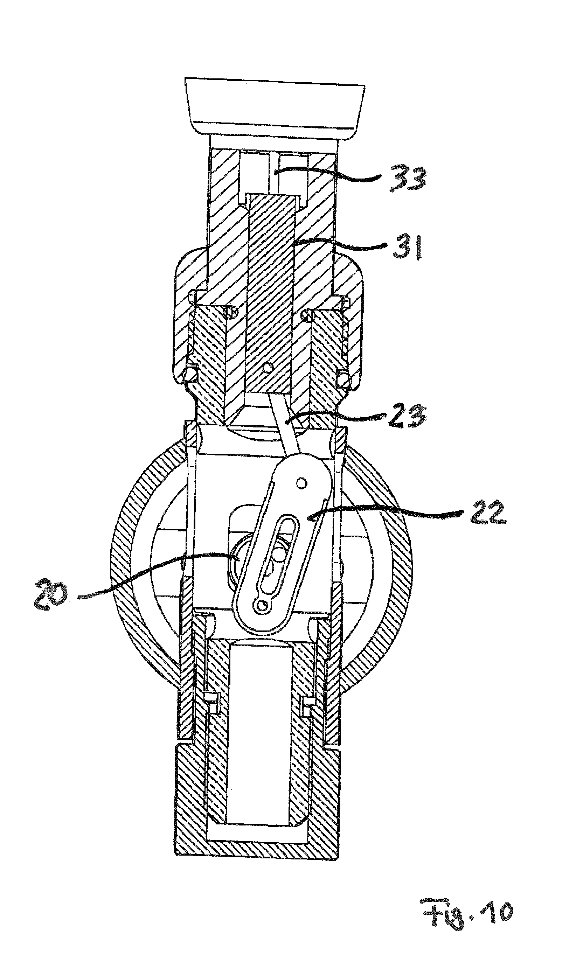

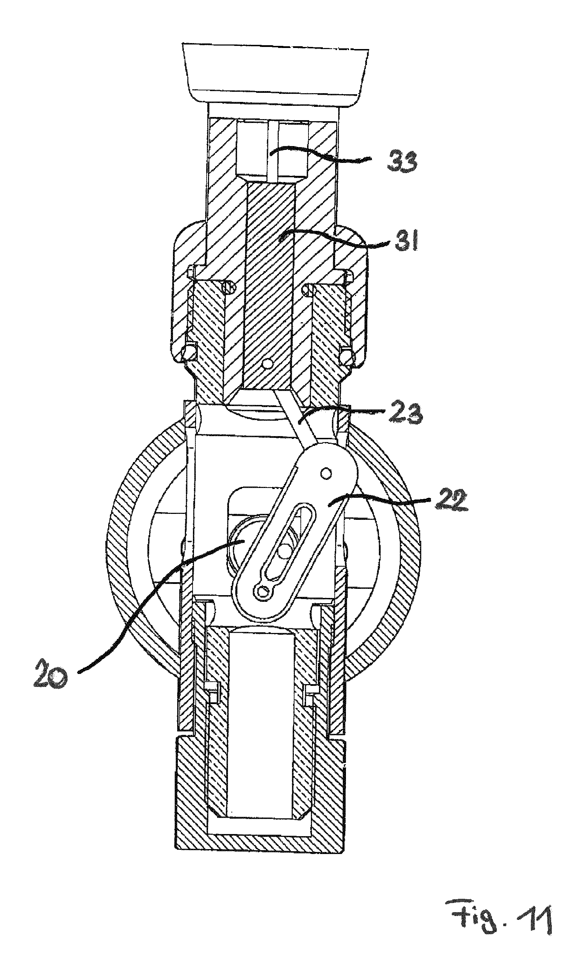

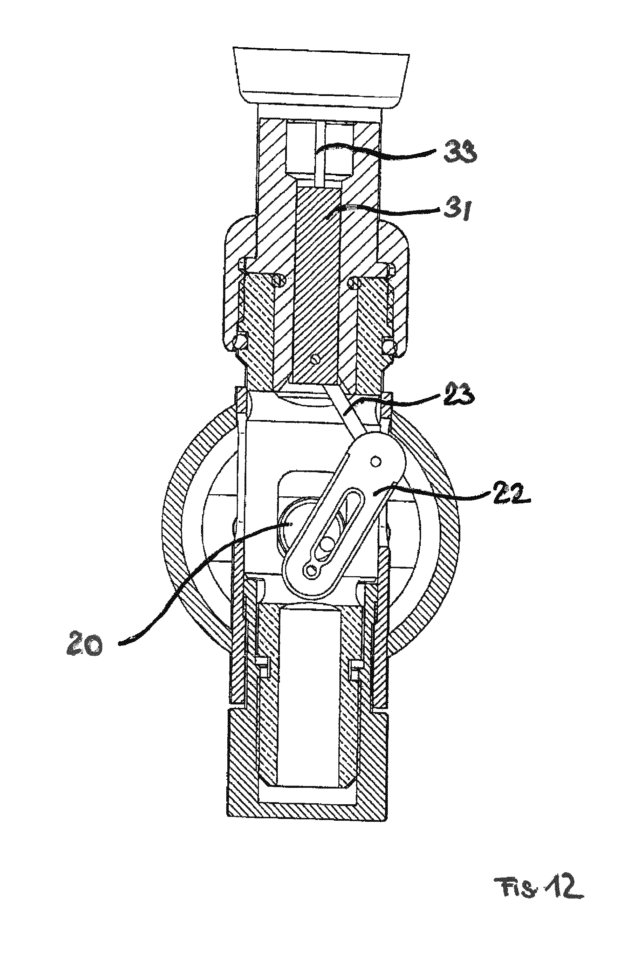

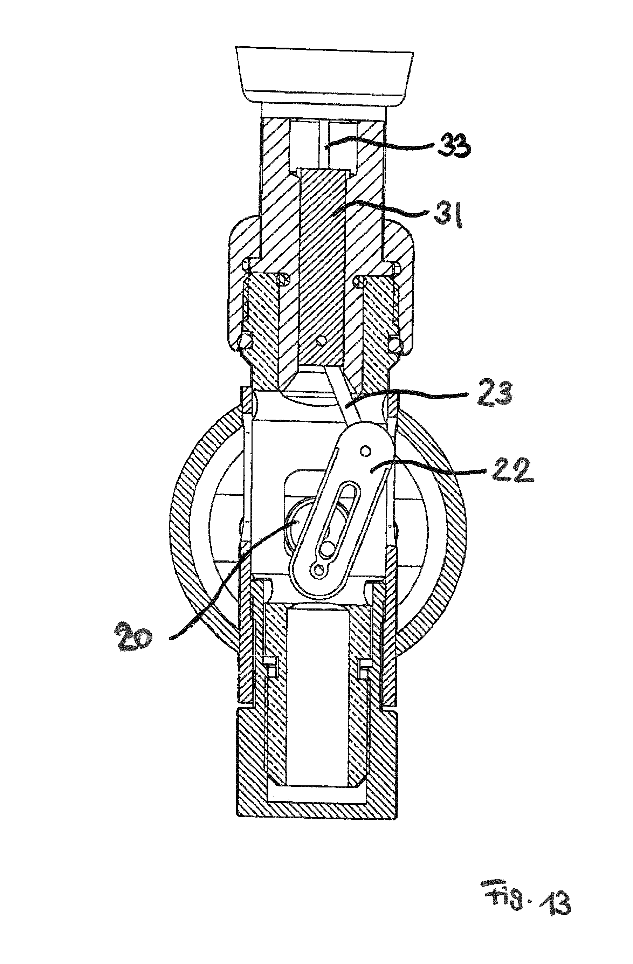

An die Antriebswelle koppelt eine Kurbeleinrichtung 20, die ihrerseits funktionell mit einer Reihenanordnung 21 von Pleuelelementen verbunden ist. Die Reihenanordnung 21 weist ein proximales Pleuelelement 22 auf, welches gelenkig mit einem distalen Pleuelelement 23 verbunden ist. Das proximale Pleuelelement 22 weist eine Verschiebe- oder Gleitlagerung 24 auf, bei der in einer Pleuelelementführung 25 ein Stift 26 verschiebbar und drehbar lagert, welcher Teil der Kurbeleinrichtung 20 ist. Das proximale Pleuelelement 22 ist an einer Gehäuselagerung 27 schwenkbar aufgenommen. Beim Drehen der Kurbeleinrichtung 20 auf-grund der von der Antriebseinrichtung bereitgestellten rotierenden Antriebsbewegung schwenkt das proximale Pleuelelement 22 um die von der Gehäuselagerung 27 bereitgestellte Schwenkachse, wobei sich hierbei der Stift 26 in der Pleuelelementführung 25 bewegt. Dieses ergibt sich insbesondere auch aus den verschiedenen Betriebsstellungen, die in den Fig. 5 bis 13 dargestellt sind, welche Draufsichten des Abschnittes des Handgerätes 1 aus den Fig. 3 und 4 zeigen.

Das proximale Pleuelelement 22 und das distale Pleuelelement 23 sind über eine Schwenkverbindung 29 gekoppelt, wie dieses auch mit Hilfe einer weiteren Schwenkverbindung 30 zwischen dem distalen Pleuelelement 23 und einem Kolben- oder Führungsbauteil 31 der Fall ist, welches in einer axialen Gehäuseführung 32 angeordnet ist. Das proximale und das distale Pleuelelement 22, 23 sind Bestandteil des Wandlungsmechanismus, mit dem die rotierende Antriebsbewegung in eine axial ausgerichtete Antriebsbewegung (Antriebskraft) umgewandelt wird, die dazu führt, dass das Kolben- oder Führungselement 31 in der Gehäuseführung 32 vor und zurück bewegt wird.

Das Kolbenelement 31 ist über ein Zwischenstück 33 mit einem Nadelschaft (nicht dargestellt) im Stechmodul verbunden, an dem die zum lokalen Aufstechen der Haut vorgesehenen Stechnadeln oder nur eine Stechnadel gelagert sind.

Bei der dargestellten Ausführungsform in den Fig. 3 und 4 ist die Gehäuselagerung 27 hinsichtlich einer gedachten Verbindung zwischen der Kurbeleinrichtung 20 und dem Kolbenelement 31 rückseitig angeordnet. Die Gehäuselagerung 27 ist auf einem Innenbauteil 34 gebildet oder hiermit verbunden, welches in axialer Richtung verlagerbar in dem Gehäuse 2 aufgenommen ist. Die Axialverschiebung erfolgt mittels Drehen des von außen zugänglichen Einstellbauteils 35. Auf diese Weise wird der Abstand zwischen der Gehäuselagerung 27 und der Drehachse der Kurbeleinrichtung 20 verändert, was schließlich die Hubbewegung des Kolbenbauteils 31 verändert.

Die Fig. 5 bis 13 zeigen verschiedene Stellungen für das proximale Pleuelelement 22, das distale Pleuelelement 23 sowie das Kolbenbauteil 31 im Verlauf einer vollständigen Umdrehung der Kurbeleinrichtung 20 im Uhrzeigersinn.

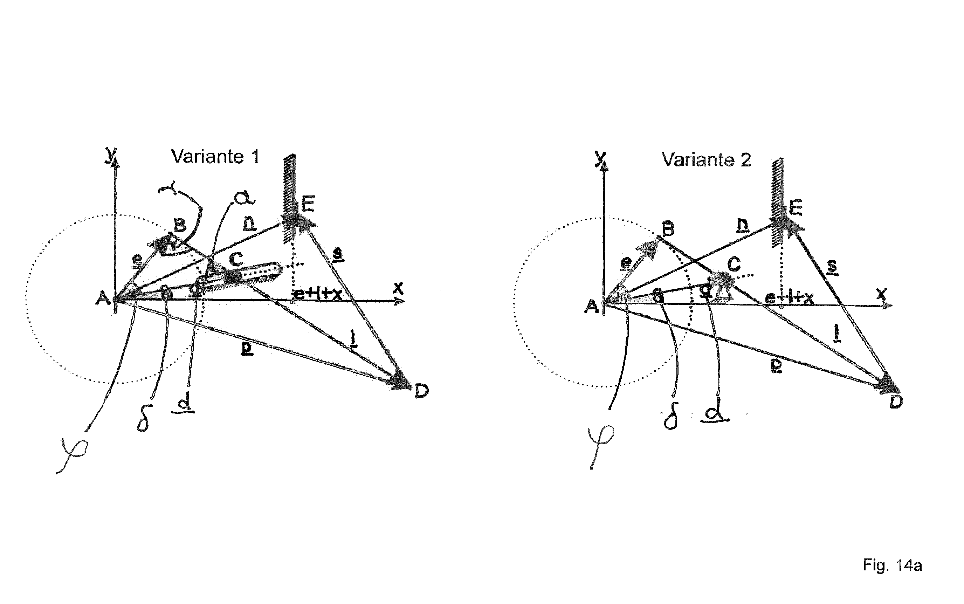

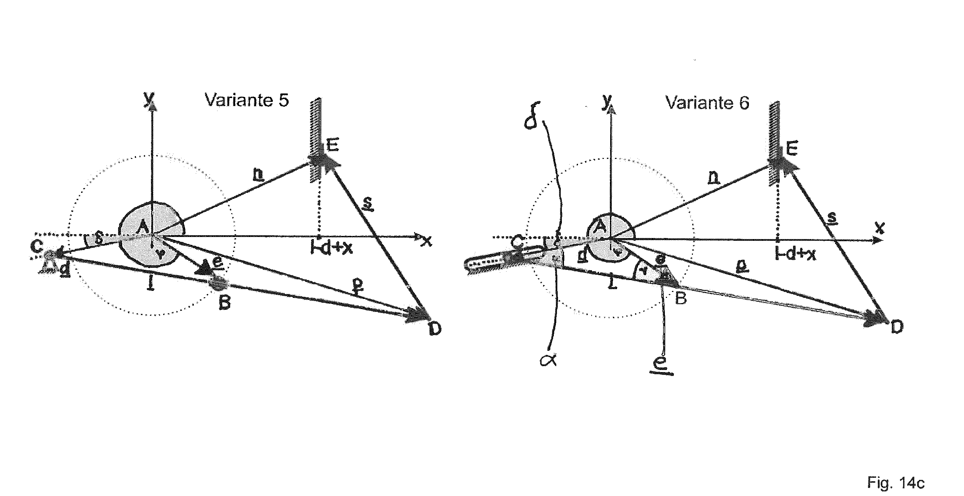

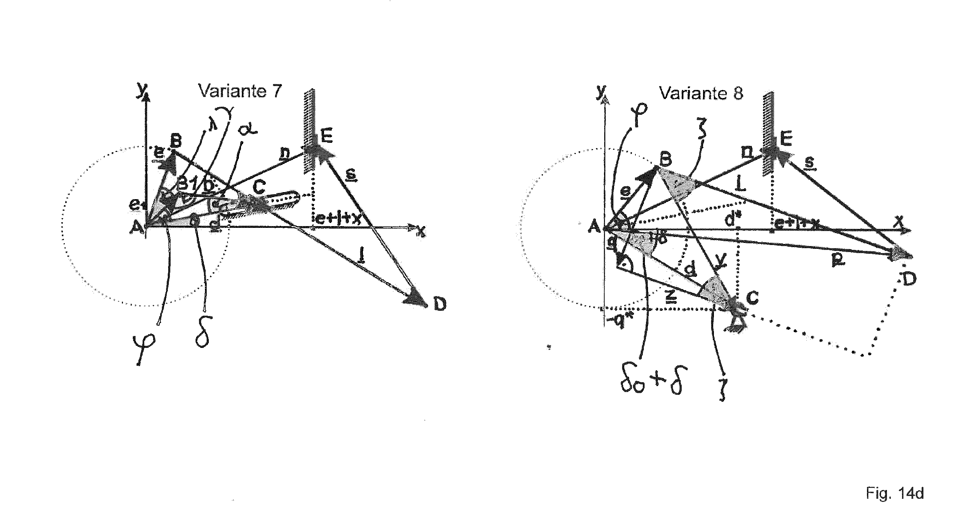

Fig. 14a bis 14d zeigen schematische Darstellungen von Beispielen 14.1 bis 14.8 (Varianten 1 bis 8) für Ausgestaltungen des Wandlungsmechanismus mit der Reihenanordnung 21 von Pleuelelementen, die an die Kurbeleinrichtung 20 koppeln und so die drehende Antriebsbewegung in eine axial ausgerichtete Antriebsbewegung wandeln.

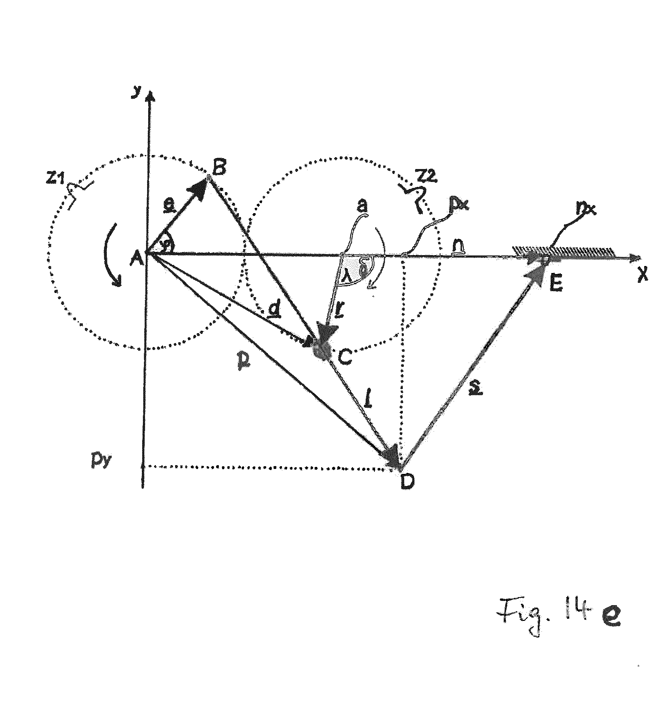

Fig. 14e zeigt eine schematische Darstellungen einer weiteren Ausführungsform (Beispiel 14.9 - Variante 9) eines Wandlungsmechanismus, welcher an eine Kurbeleinrichtung koppelt.

Nachfolgend werden die Ausgestaltungen 14.1 bis 14.8 (Varianten 1 bis 8) aus den Fig. 14a bis 14d näher beschrieben:

Beispiel 14.1: Ein Gleitstift C, der mittels einer Gehäuselagerung gebildet ist, ist fest mit dem proximalen Pleuel verbunden und gleitet in einer Längsnut hierin, die unter einem Winkel $\delta$ zur x-Achse verläuft.

Beispiel 14.2: Der Gleitstift C ist ortsfest und gleitet in einer Nut in der Pleuellängsachse.

Beispiel 14.3: Der Gleitstift C ist ortsfest und drehbar mit dem proximalen Pleuel verbunden, eine Antriebskurbel B gleitet auf einer rückwärtigen Verlängerung der Pleuellängsachse in einer Nut.

Beispiel 14.4: Der Gleitstift C ist ortsfest und drehbar mit dem proximalen Pleuel verbunden, die Motorwelle der Antriebskurbel A gleitet in eine Nut, die unter einem Winkel $\delta$ zur x-Achse verläuft.

Beispiel 14.5: Ähnlich Beispiel 14.3 - die Antriebskurbel gleitet jedoch zwischen den beiden Pleuelaugen C und D.

Beispiel 14.6: Ähnlich Beispiel 14.1 - der Gleitstift C befindet sich jedoch außerhalb der Verbindungslinie beider Pleuelaugen B und D.

Beispiel 14.7: Kombination aus den Beispielen 14.1 und 14.2, wobei der Abstand des Gleitstifts C durch eine zusätzliche Kurbel ohne oder mit festem oder zeitveränderlichem Phasenwinkel $\lambda$ bestimmt wird.

Beispiel 14.8: Ähnlich zu Beispiel 14.2 - es existiert jedoch eine gewisse Schränkung q (Parallelversatz) zwischen der Pleuelaugenverbindungslinie B und D und der Gleitstift-Gleitnut C im Pleuel.

Das Beispiel 14.1 weist einen pleuelfesten Gleitstift auf, der in einer Nut im Gehäusegleitet. Der Stell- oder Gleitstift C in den Fig. 14a bis 14d ist im Abstand $l_1$ vom unteren Pleuelauge B entfernt fest mit dem Pleuel verbunden. Der Stift gleitet in einer Nut in der Wand des Gehäuses, die bezüglich der Abszisse unter einem Höhenwinkel $\delta$ in Richtung AC verläuft.

Der Winkel $\delta$ sei einstellbar, aber als zeitlich konstant anzusehen. Er stellt die Variable für die Hubeinstellung dar. Das Bezugskoordinatensystem hat seinen Ursprung in der Symmetrieachse der Motorwelle, dem Punkt A. Der Vektor e stellt die Verbindung zwischen dem Punkt A und dem Mittelpunkt des unteren Pleuelauges B dar. Sein Betrag sei e und zeitlich konstant. Der Vektor d stellt die Koordinaten des Gleitstifts C dar, sein Betrag sei d und zeitlich veränderlich. Er schließt den Höhenwinkel $\delta$ mit der Abszisse ein. Der Vektor l verbindet das untere Pleuelauge B mit dem oberen Pleuelauge D über den Gleitstift C hinweg. Der Betrag des Vektors l sei l. Der Abstand BC ist dabei ein Teilstück des Vektors l und wird mit $l_1$ bezeichnet. Der Vektor p zeigt auf die Koordinaten des oberen Pleuelauges D. Der Vektor s stellt die Längsachse der Schubstange DE dar, welche das obere Pleuelauge D mit dem Kolben E verbindet. Die Länge der Schubstange wurde mit $l_s$ bezeichnet. Der Kolben, welcher die Nadel aufnehmen soll, besitzt eine feste x-Koordinate, lediglich die y-Koordinate ist zeitvariant. Der Vektor n zeigt dabei auf die Koordinaten des Kolbens im Koordinatensystem (vgl. Fig. 14a bis 14d).

Die Abhängigkeit des Kurbelwinkels $\varphi(t)$ von der Zeit t wird vereinfachend als linear angenommen und damit folgende Beziehung zur konstanten Winkelgeschwindigkeit $\omega_0$ erhalten, wobei die geforderte Stechfrequenz mit f, die daraus abgeleitete Periodendauer mit T bezeichnet wird.

$$\varphi(t) = 2\pi \cdot f \cdot t = \frac{2\pi}{T} \cdot t = \omega_0 \cdot t \quad \text{mit } \omega_0 = \text{const.} \tag{0-1}$$

Es lässt sich der zeitabhängige Kurbelvektor e schreiben als

$$\vec{e}(t) = \underbrace{\begin{bmatrix} \cos(\varphi(t)) & -\sin(\varphi(t)) \\ \sin(\varphi(t)) & \cos(\varphi(t)) \end{bmatrix}}_{\text{2D-Drehmatrix}} \cdot \underbrace{\begin{bmatrix} e \\ 0 \end{bmatrix}}_{\substack{\text{Vektor} \\ e_0}} = e \cdot \begin{bmatrix} \cos(\varphi(t)) \\ \sin(\varphi(t)) \end{bmatrix} \tag{0-2}$$

mit e = const.

Der in seinem Betrag zeitveränderliche Vektor d, welcher vom Koordinatenursprung A zum Gleitstift C zeigt, ergibt sich unter Anwendung des Sinussatzes für beliebige Dreiecke und der Beziehung für die Innenwinkelsumme zu:

$$\vec{d}(t,\delta) = \underbrace{\sqrt{e^2 + l_1^2 - 2 \cdot e \cdot l_1 \cdot \cos(\gamma(t))}}_{\substack{= |\vec{d}(t,\delta)|; \text{ folgt aus dem Kosinussatz für} \\ \text{beliebige Dreiecke}}} \cdot \begin{bmatrix} \cos(\delta) \\ \sin(\delta) \end{bmatrix} \tag{0-3}$$

mit e, l, $\delta$ = const.
mit

$$\gamma(t) = \pi - \left[ \underbrace{\arcsin\left( \underbrace{\frac{e}{l_1} \cdot \sin(\varphi(t) - \delta)}_{= \sin(\alpha), \text{ Sinussatz}} \right)}_{= \alpha, \text{ Innenwinkel 1}} + \underbrace{(\varphi(t) - \delta)}_{\text{Innenwinkel 2}} \right]$$

folgt aus Innenwinkelsumme im beliebigen Dreieck

Somit lässt sich der Vektor l unter Verwendung seines Betrags l und des mit $l_1$ bekannten festen Abstands BC errechnen:

$$\vec{l}(t,\delta) = l \cdot \underbrace{\frac{\vec{d}(t,\delta) - \vec{e}(t)}{l_1}}_{\substack{= \text{normierter Vektor} \\ \text{in Pleuelrichtung}}} \quad \text{mit } l, l_1, \delta = \text{const.} \tag{0-4}$$

Es ergibt sich der Ortsvektor p des oberen Pleuelauges D im Koordinatensystem:

$$\vec{p}(t,\delta) = \vec{l}(t,\delta) + \vec{e}(t) \qquad (0\text{-}5)$$

Der Lagevektor s der Schubstange bildet mit der Vertikalen durch den Punkt E und mit der Horizontalen durch den Punkt D ein rechtwinkliges Dreieck und kann deshalb mit dem Satz des Pythagoras unter Verwendung der bekannten Länge $l_s$ und der festen x-Position des Punkts E ermittelt werden, wobei im Folgenden $p_x(t)$ und $p_y(t)$ die zeitveränderlichen Komponenten des Pleuelvektors p und x einen frei wählbaren Wert für den horizontalen Versatz des Kolbens von der x-Bezugslage e + l darstellen:

$$\vec{s}(t,\delta) = \begin{bmatrix} e + l + x - p_x(t,\delta) \\ \sqrt{l_s^2 - \left(e + l + x - p_x(t,\delta)\right)^2} \end{bmatrix} \qquad (0\text{-}6)$$

mit e, l, x, $l_s$ = const.

Als Summe der Vektoren p und s ergibt sich schließlich der Vektor n des Kolbens im Koordinatensystem:

$$\vec{n}(t,\delta) = \vec{s}(t,\delta) + \vec{p}(t,\delta)$$

$$\vec{n}(t,\delta) = \begin{bmatrix} e + l + x \\ \sqrt{l_s^2 - \left(e + l + x - p_x(t,\delta)\right)^2} + p_y(t,\delta) \end{bmatrix} \qquad (0\text{-}7)$$

mit e, l, x, $l_s$ = const.

Für die Geschwindigkeit des Kolbens gilt:

$$\vec{v}_{\text{Kolben}}(t_i,\delta) = \dot{\vec{n}}(t_i,\delta) = \lim_{\Delta t \to 0} \frac{\vec{n}(t_i + \Delta t, \delta) - \vec{n}(t_i,\delta)}{\Delta t}$$

$$\vec{v}_{\text{Kolben}}(t_i,\delta) \approx \underbrace{\frac{\vec{n}(t_i + \Delta t, \delta) - \vec{n}(t_i,\delta)}{\Delta t}}_{\text{für ein sehr kleines } \Delta t} \qquad (0\text{-}8)$$

Bei hoher zeitlicher Auflösung und damit sehr kleinem $\Delta t$ kann für die Ermittlung der Geschwindigkeit mit ausreichender Genauigkeit mit dem Differenzenquotienten gerechnet werden.

Es kann günstig sein, die Forderung nach einer linearen Gleitnut in Richtung AC sowie einer konstanten Winkelgeschwindigkeit $\omega_0$ aufzugeben. Dies wurde jedoch nicht untersucht.

Bei dem Beispiel 14.2 ist Folgendes vorgesehen: Pleuellängsachse gleitet in drehbar gelagerter, wandfester Hülse.

Für die Entwicklung eines Handgerätes mit möglichst hoher Einstechgeschwindigkeit und genügend langer Dosierphase sowie einer Einstellmöglichkeit für den hubabhängigen Nadelherausstand wurde ein Handgerät gemäß der Ausführung 14.2 aufgebaut, bei dem das untere Pleuelauge durch einen rotierenden Exzenter bewegt wird und zwischen unterem und oberem Pleuelauge ein zum Gehäuse feststehender Umlenkpunkt in einer Längsnut im Pleuel gleitet. Vom oberen Pleuelauge wird die Bewegung schließlich über eine Schubstange auf den Abtriebskolben oder -stößel übertragen. Zusätzlich fällt die Stechgeschwindigkeit beim Einstechvorgang bezüglich des Wegs erst spät ab, was günstig für das Erreichen geringer Stechtiefen ist.

Um die formale Abhängigkeit der Kolbenbewegung vom zeitveränderlichen Antriebsdrehwinkel $\varphi(t)$ und vom zeitinvarianten, einstellbaren Höhenwinkel $\delta$ herstellen zu können, wurde zunächst die Kolbenbewegung vektoriell beschrieben. Für den Winkel $\varphi(t)$ kann jede Zeitabhängigkeit angenommen werden, in der Beziehung (0-9) wird beispielhaft ein linearer zeitlicher Zusammenhang, resultierend aus einer konstanten Motordrehzahl, dargestellt.

$$\varphi(t) = 2\pi \cdot f \cdot t = \frac{2\pi}{T} \cdot t = \omega_0 \cdot t \quad \text{mit} \quad \omega_0 = \text{const.} \tag{0-9}$$

Es lässt sich der Kurbelvektor e als Funktion des zeitabhängigen Drehwinkels $\varphi(t)$ und der konstanten Kurbellänge e schreiben:

$$\vec{e}(t) = \underbrace{\begin{bmatrix} \cos(\varphi(t)) & -\sin(\varphi(t)) \\ \sin(\varphi(t)) & \cos(\varphi(t)) \end{bmatrix}}_{\text{2D-Drehmatrix}} \cdot \underbrace{\begin{bmatrix} e \\ 0 \end{bmatrix}}_{\substack{\text{Vektor} \\ e_0}} = e \cdot \begin{bmatrix} \cos(\varphi(t)) \\ \sin(\varphi(t)) \end{bmatrix} \tag{0-10}$$

In Fig. 14.2 stellt der Punkt C einen zylindrischen Gleitstift dar, der in einer Langnut im Pleuel BD gleitet, welche in der gedachten Pleuellängsachse liegt. C ist fest mit einem um die Motorachse drehbaren Gleitstiftträger (GST) verbunden, dessen Drehwinkel $\delta$ zeitlich konstant gehalten und nur zur Einstellung des Nadelherausstands verändert wird.

Das Bezugskoordinatensystem hat seinen Ursprung wieder in der Dreh- und Symmetrieachse der Motorwelle, dem Punkt A. Der Vektor e stellt die Verbindung zwischen dem Punkt A und dem Mittelpunkt B des unteren Pleuelauges dar. Sein Betrag sei e und zeitlich konstant. Der Vektor l verbindet das untere Pleuelauge B mit dem oberen Pleuelauge D. Der Betrag von l sei l. Der Vektor d stellt die Koordinaten des Gleitstifts C dar, sein Betrag sei d und zeitlich konstant. Er schließt den Höhenwinkel $\delta$ mit der Abszisse ein. Dieser stellt, wie bereits oben beschrieben, die Variable für die Einstellung des Nadelheraus- oder Nadelüberstands in Bezug auf die vorderer Gehäuseöffnung dar. Der Vektor p zeigt auf die Koordinaten des oberen Pleuelauges D. Der Vektor s stellt die Längsachse der Schubstange DE dar, welche das obere Pleuelauge D mit dem Kolben E verbindet. Der Kolben E, welcher die Nadel aufnehmen soll, besitzt eine feste x-Koordinate, lediglich die y-Koordinate ist zeitvariant. Der Vektor n zeigt dabei auf die Koordinaten des Kolbens E bezüglich des Koordinatensystems.

Der hier zeitlich konstante Vektor d, welcher vom Koordinatenursprung A zum Gleitstift C zeigt, ergibt sich zu:

$$\vec{d}(\delta) = d \cdot \begin{bmatrix} \cos(\delta) \\ \sin(\delta) \end{bmatrix} \quad \text{mit} \quad d, \delta = \text{const.} \tag{0-11}$$

Der zeitabhängige Vektor l wird nachfolgend unter Verwendung seines Betrages l errechnen:

$$\vec{l}\left(t,\delta\right) = l \cdot \underbrace{\frac{\vec{d}(\delta) \; - \; \vec{e}(t)}{\left|\vec{d}(\delta) \; - \; \vec{e}(t)\right|}}_{\substack{= \text{ normierter Vektor} \\ \text{in Pleuelrichung}}} \quad \text{mit } l = \text{const.}$$

(0-12)

Es ergibt sich der Ortsvektor p des oberen Pleuelauges D im Koordinatensystem:

$$\vec{p}\left(t,\delta\right) = \vec{l}\left(t,\delta\right) \; + \; \vec{e}\left(t\right)$$

(0-13)

Der Lagevektor s der Schubstange DE bildet mit der Vertikalen durch den Punkt E und mit der Horizontalen durch den Punkt D ein rechtwinkliges Dreieck und kann deshalb mittels Satz des Pythagoras unter Verwendung der vorgegebenen Länge der Schubstange ls und der bekannten x-Position des Punkts E ermittelt werden, wobei im Folgenden px(t) und py(t) die zeitveränderlichen Komponenten des Pleuelvektors p, und x einen frei wählbaren Wert für den horizontalen Versatz des Kolbens E von der x0-Bezugslage e + l darstellen:

$$\vec{s}\left(t,\delta\right) = \left[\begin{array}{c} e \; + \; l \; + \; x \; - \; p_x\left(t,\delta\right) \\ \sqrt{l_s^2 \; - \; \left(e \; + \; l \; + \; x \; - \; p_x\left(t,\delta\right)\right)^2} \end{array}\right]$$

(0-14)

mit e, l, x, $l_s$ = const.

Als Summe der Vektoren p und s ergibt sich schließlich der auf den Kolben E zeigende Vektor n im Koordinatensystem:

$$\vec{n}\left(t,\delta\right) = \vec{s}\left(t,\delta\right) \; + \; \vec{p}\left(t,\delta\right)$$

$$\vec{n}\left(t,\delta\right) = \left[\begin{array}{c} e \; + \; l \; + \; x \\ \sqrt{l_s^2 \; - \; \left(e \; + \; l \; + \; x \; - \; p_x\left(t,\delta\right)\right)^2} \; + \; p_y\left(t,\delta\right) \end{array}\right]$$

(0-15)

mit e, l, x, $l_s$ = const.

Um die Geschwindigkeit des Kolbens E zu erhalten, wird die Zeitableitung des Nadelvektors gebildet:

$$\vec{v}_{\text{Kolben}}\left(t_i,\delta\right) = \dot{\vec{n}}\left(t_i,\delta\right) = \lim_{\Delta t \to 0} \frac{\vec{n}\left(t_i + \Delta t,\delta\right) \; - \; \vec{n}\left(t_i,\delta\right)}{\Delta t}$$

(0-16)

Bei hoher zeitlicher Auflösung und damit sehr kleinem $\Delta t$ kann für die Ermittlung der Geschwindigkeit mit ausreichender Genauigkeit mit dem Differenzenquotienten gerechnet werden.

$$\vec{v}_{Kolben}\left(t_i, \delta\right) \approx \underbrace{\frac{\vec{n}\left(t_i + \Delta t, \delta\right) - \vec{n}\left(t_i, \delta\right)}{\Delta t}}_{\text{für ein sehr kleines } \Delta t} \qquad (0\text{-}17)$$

Die Variation des Höhenwinkels $\delta$ stellt eine Möglichkeit dar, den Nadelherausstand und gleichzeitig den Hub zu ändern.

Bei dem Beispiel 14.3 ist Folgendes vorgesehen: Pleuelfester Gleitstift ist drehbar wandfest gelagert, Kurbel gleitet auf Pleuellängsachse. Die Eigenschaft einer Kurbel mit Gleithülse B kann bei kleiner Exzentrizität e beispielsweise auch durch ein Langloch-Pleuelauge mit parallelen Gleitflächen gebildet werden, in welchem eine seitlich spielfrei passende Zylinderscheibe mit einer Exzentrizität e um den Punkt A gleitend rotiert, wobei die Lastseite entsprechend wechselt.

Das Bezugskoordinatensystem hat seinen Ursprung in der Symmetrieachse der Motorwelle, dem Punkt A. Der Vektor e stellt die Verbindung zwischen dem Punkt A und dem Mittelpunkt der Hülse B auf der Kurbelscheibe dar. Sein Betrag sei e und zeitlich konstant. Der Vektor l verbindet den Gleitstift C mit dem oberen Pleuelauge D. Der Betrag von l sei berechnet als die Differenz aus l und $l_1$ und zeitlich konstant. Der Vektor d stellt die Koordinaten des Gleitstifts C dar. Er ist zeitlich konstant, sein Betrag sei d. Er schließt den Höhenwinkel $\delta$ mit der Abszisse ein. Der Winkel $\delta$ sei einstellbar, aber ebenfalls als zeitlich konstant anzusehen. Er stellt die Variable für die Hubverstellung dar. Der Vektor p zeigt auf die Koordinaten des oberen Pleuelauges D. Der Vektor s stellt die Längsachse der Schubstange DE dar, welche das obere Pleuelauge D mit dem Kolben E verbindet. Der Kolben, welcher die Nadel aufnehmen soll, besitzt eine feste x-Koordinate, lediglich die y-Koordinate ist zeitvariant. Der Vektor n zeigt dabei auf die Koordinaten des Kolbens bezüglich des Koordinatensystems.

Somit lässt sich der Vektor l unter Verwendung der bekannten Werte l und $l_1$ errechnen:

$$\vec{l}\left(t, \delta\right) = \left(1 - l_1\right) \cdot \underbrace{\frac{\vec{d}\left(\delta\right) - \vec{e}\left(t\right)}{\left|\vec{d}\left(\delta\right) - \vec{e}\left(t\right)\right|}}_{\substack{= \text{ normierter Vektor} \\ \text{in Pleuelrichung}}} \quad \text{mit } l, l_1 = \text{const.} \qquad (0\text{-}18)$$

Es ergibt sich der Ortsvektor p des oberen Pleuelauges D im Koordinatensystem:

$$\vec{p}\left(t, \delta\right) = \vec{l}\left(t, \delta\right) + \vec{d}\left(\delta\right) \qquad (0\text{-}19)$$

Der Lagevektor s der Schubstange bildet mit der Vertikalen durch den Punkt E und mit der Horizontalen durch den Punkt D ein rechtwinkliges Dreieck und kann deshalb mit dem Satz des Pythagoras unter Verwendung der bekannten Länge $l_s$ und der festen x-Position des Punkts E ermittelt werden, wobei im Folgenden $p_x(t)$ und $p_y(t)$ die zeitveränderlichen Komponenten des Pleuelvektors p und x einen frei wählbaren Wert für den horizontalen Versatz des Kolbens von der $(x = 0)$-Bezugslage d + $(l - l_1)$ darstellen:

$$\vec{s}\left(t,\delta\right) = \left[\begin{array}{c} d + 1 - l_1 + x - p_x\left(t,\delta\right) \\ \sqrt{l_s^2 - \left(d + 1 - l_1 + x - p_x\left(t,\delta\right)\right)^2} \end{array}\right] \qquad (0\text{-}20)$$

mit e, l, $l_1$, x, $l_s$ = const.

Als Summe der Vektoren p und s ergibt sich schließlich der Vektor n des Kolbens im Koordinatensystem:

$$\vec{n}\left(t,\delta\right) = \vec{s}\left(t,\delta\right) + \vec{p}\left(t,\delta\right)$$

$$\vec{n}\left(t,\delta\right) = \left[\begin{array}{c} d + 1 - l + x \\ \sqrt{l_s^2 - \left(d + 1 - l_1 + x - p_x t\left(,\delta\right)\right)^2} + p_y\left(t,\delta\right) \end{array}\right] \qquad (0\text{-}21)$$

mit e, l, x, $l_s$ = const.

Der nicht erneut dargestellte Ortsvektor e sowie der Geschwindigkeitsvektor v des Kolbens und der Winkelvektor $\varphi$ lassen sich wie bei dem Beispiel 14.1 ermitteln, siehe dort.

Bei dem Beispiel 14.4 ist Folgendes vorgesehen: Pleuelfester Gleitstift ortsfest drehbar in Gehäusewand, Motorwelle gleitet in Gehäusenut.

Die Nut in der Gehäusewand sei verstellbar und verlaufe unter einem Winkel $\delta$ bezüglich der negativen Abszisse. Das Bezugskoordinatensystem hat seinen Ursprung im Gleitstift, dem Punkt C. Der Vektor e stellt die Verbindung zwischen dem Punkt A und dem unteren Pleuelauge, Punkt B, dar. Sein Betrag sei e und zeitlich konstant. Der Vektor l stellt die schematische Reduktion des Pleuels dar und verbindet den Punkt B über den Gleitstift C hinweg mit dem Punkt D. Der Betrag von l sei l, der vom Abstand BC sei $l_1$, beide seien zeitlich konstant. Der Vektor d stellt die invertierten Koordinaten der Motorwelle A dar und besitzt somit positive Komponenten, sein Betrag sei d und zeitlich veränderlich. Er schließt den Höhenwinkel $\delta$ mit der Abszisse ein. Der Winkel $\delta$ sei einstellbar, aber als zeitlich konstant anzusehen. Er stellt die Variable für die Hubverstellung dar. Der Vektor p zeigt auf die Koordinaten des oberen Pleuelauges D. Der Vektor s stellt die Längsachse der Schubstange DE dar, welche das obere Pleuelauge D mit dem Kolben E verbindet. Der Kolben, welcher unter anderem die Nadel aufnehmen soll, besitzt eine feste x-Koordinate, lediglich die y-Koordinate ist zeitvariant. Der Vektor n zeigt dabei auf die Koordinaten des Kolbens bezüglich des Koordinatensystems.

Wegen der Ähnlichkeit zur Ausführung 14.1 werden hier lediglich die abweichenden Vektoren dargestellt. Die Verhältnisse am Dreieck ABC sind unverändert geblieben. Jedoch unterscheidet sich der Vektor p:

$$\vec{p}\left(t,\delta\right) = \left(1 - l_1\right)\cdot\underbrace{\frac{\vec{d}\left(t,\delta\right) - \vec{e}\left(t\right)}{l_1}}_{\substack{= \text{normierter Vektor} \\ \text{in Pleuelrichtung}}} \quad \text{mit } l, l_1 = \text{const.} \qquad (0\text{-}22)$$

Damit ergeben sich auch Änderungen an den von p abhängigen Vektoren. Der Lagevektor s der Schubstange bildet mit der Vertikalen durch den Punkt E und mit der Horizontalen durch den Punkt D ein rechtwinkliges Dreieck und kann deshalb mit dem Satz des Pythagoras unter Verwendung der bekannten Länge $l_s$ und der festen x-Position des Punktes E ermittelt werden, wobei im Folgenden $p_x(t)$ und $p_y(t)$ die zeitveränderlichen Komponenten des Pleuelvektors p und x einen frei wählbaren Wert für den horizontalen Versatz des Kolbens von der x-Bezugslage l - l$_1$ darstellen:

$$\vec{s}(t, \delta) = \begin{bmatrix} 1 - l_1 + x - p_x(t, \delta) \\ \sqrt{l_s^2 - (1 - l_1 + x - p_x(t, \delta))^2} \end{bmatrix} \qquad (0\text{-}23)$$

mit l, l$_1$, x, l$_s$ = const.

Als Summe der Vektoren p und s ergibt sich schließlich der Vektor n des Kolbens im Koordinatensystem:

$$\vec{n}(t, \delta) = \vec{s}(t, \delta) + \vec{p}(t, \delta)$$

$$\vec{n}(t, \delta) = \begin{bmatrix} 1 - l_1 + x \\ \sqrt{l_s^2 - (1 - l_1 + x - p_x t(, \delta))^2} + p_y(t, \delta) \end{bmatrix} \qquad (0\text{-}24)$$

mit l, l$_1$, x, l$_s$ = const.

Die nicht erneut dargestellten Ortsvektoren e, l und d sowie der Geschwindigkeitsvektor v des Kolbens und der Winkelvektor $\varphi$, lassen sich wie bei der Ausführung 1 ermitteln, siehe dort.

Bei dem Beispiel 14.5 liegt die Fesselung des Pleuels außerhalb der Antriebs-Abtriebs-Verbindung. In allen anderen Punkten gilt das zu Ausführung 14.3 Gesagte entsprechend.

Der hier zeitlich konstante Vektor d, welcher vom Koordinatenursprung A zum Gleitstift C zeigt, ergibt sich zu:

$$\vec{d}(\delta) = d \cdot \begin{bmatrix} -\cos(\delta) \\ -\sin(\delta) \end{bmatrix} \quad \text{mit } d, \delta = \text{const.} \qquad (0\text{-}25)$$

Somit lässt sich der Vektor l unter Verwendung seines Betrags l errechnen:

$$\vec{l}(t, \delta) = 1 \cdot \underbrace{\frac{\vec{e}(t) - \vec{d}(\delta)}{|\vec{e}(t) - \vec{d}(\delta)|}}_{\substack{= \text{normierter Vektor} \\ \text{in Pleuelrichtung}}} \quad \text{mit } 1 = \text{const.} \qquad (0\text{-}26)$$

Es ergibt sich der Ortsvektor p des oberen Pleuelauges D im Koordinatensystem:

$$\vec{p}(t, \delta) = \vec{l}(t, \delta) \; + \; \vec{d}(\delta) \qquad\qquad (0\text{-}27)$$

Der Lagevektor s der Schubstange bildet mit der Vertikalen durch den Punkt E und mit der Horizontalen durch den Punkt D ein rechtwinkliges Dreieck und kann deshalb mit dem Satz des Pythagoras unter Verwendung der bekannten Länge $l_s$ und der festen x-Position des Punkts E ermittelt werden, wobei im Folgenden $p_x(t)$ und $p_y(t)$ die zeitveränderlichen Komponenten des Pleuelvektors p und x einen frei wählbaren Wert für den horizontalen Versatz des Kolbens von der x-Bezugslage l - d darstellen:

$$\vec{s}(t, \delta) = \begin{bmatrix} l \, - \, d \, + \, x \, - \, p_x(t, \delta) \\ \sqrt{l_s^2 \, - \, \left(l \, - \, d \, + \, x \, - \, p_x(t, \delta)\right)^2} \end{bmatrix} \qquad (0\text{-}28)$$

mit d, l, x, $l_s$ = const.

Als Summe der Vektoren p und s ergibt sich schließlich der Vektor n des Kolbens im Koordinatensystem:

$$\vec{n}(t, \delta) = \vec{s}(t, \delta) \; + \; \vec{p}(t, \delta)$$

$$\vec{n}(t, \delta) = \begin{bmatrix} l \, - \, d \, + \, x \\ \sqrt{l_s^2 \, - \, \left(l \, - \, d \, + \, x \, - \, p_x(t, \delta)\right)^2} \, + \, p_y(t, \delta) \end{bmatrix} \qquad (0\text{-}29)$$

mit d, l, x, $l_s$ = const.

Der nicht erneut dargestellte Ortsvektor e sowie der Geschwindigkeitsvektor v des Kolbens und der Winkelvektor φ, lassen sich wie bei der Ausführung 14.1 ermitteln, siehe dort.

Bei dem Beispiel 14.6 liegt die Fesselung des Pleuels außerhalb der Antriebs-Abtriebs-Verbindung. In allen anderen Punkten gilt das zur Ausführung 14.1 Gesagte (vgl. Fig. 14a) Wegen der veränderten Gestalt des aufgespannten Dreiecks ABC im Vergleich zu Ausführung 14.1 müssen die Winkelbeziehungen angepasst werden.

Der in seinem Betrag zeitveränderliche Vektor d, welcher vom Koordinatenursprung A zum Gleitstift C zeigt, ergibt sich unter Anwendung des Sinussatzes für beliebige Dreiecke und der Beziehung für die Innenwinkelsumme zu:

$$\vec{d}(t, \delta) = \underbrace{\sqrt{e^2 \, + \, l_1^2 \, - \, 2 \cdot e \cdot l_1 \cdot \cos(\gamma(t))}}_{= \, |\vec{d}(t, \delta)|; \; \text{folgt aus dem Kosinussatz für beliebige Dreiecke}} \cdot \begin{bmatrix} - \cos(\delta) \\ - \sin(\delta) \end{bmatrix} \qquad (0\text{-}30)$$

mit e, l, δ = const.

$$\text{mit } \gamma(t) = \pi - \left( \underbrace{\left( \underbrace{\arcsin\left( \underbrace{\frac{e}{l_1} \cdot \sin\left(\varphi(t) - \pi - \delta\right)}_{= \sin(\alpha),\ Sinussatz} \right)}_{= \alpha,\ Innenwinkel\ 1} + \underbrace{\left(\varphi(t) - \pi - \delta\right)}_{Innenwinkel\ 2} \right)}_{folgt\ aus\ Innenwinkelsumme\ im\ beliebigen\ Dreieck} \right)$$

Somit lässt sich der Vektor I unter Verwendung seines Betrags I und des mit $l_1$ bekannten festen Abstands BC errechnen:

$$\vec{l}(t, \delta) = l \cdot \underbrace{\frac{\vec{e}(t) - \vec{d}(t, \delta)}{\left|\vec{e}(t) - \vec{d}(t, \delta)\right|}}_{\substack{= \text{normierter Vektor} \\ \text{in Pleuelrichung}}} \quad \text{mit } l, l_1 = \text{const.} \qquad (0\text{-}31)$$

Es ergibt sich der Ortsvektor p des oberen Pleuelauges D im Koordinatensystem:

$$\vec{p}(t, \delta) = \vec{l}(t, \delta) + \vec{d}(t, \delta) \qquad (0\text{-}32)$$

Der Lagevektor s der Schubstange bildet mit der Vertikalen durch den Punkt E und mit der Horizontalen durch den Punkt D ein rechtwinkliges Dreieck und kann deshalb mit dem Satz des Pythagoras unter Verwendung der bekannten Länge $l_s$ und der festen x-Position des Punkts E ermittelt werden, wobei im Folgenden $p_x(t)$ und $p_y(t)$ die zeitveränderlichen Komponenten des Pleuelvektors p und x einen frei wählbaren Wert für den horizontalen Versatz des Kolbens von der x-Bezugslage e + I darstellen:

$$\vec{s}(t, \delta) = \begin{bmatrix} l - d + x - p_x(t, \delta) \\ \sqrt{l_s^2 - \left(l - d + x - p_x(t, \delta)\right)^2} \end{bmatrix} \qquad (0\text{-}33)$$

mit e, I, x, $l_s$ = const.

Als Summe der Vektoren p und s ergibt sich schließlich der Vektor n des Kolbens im Koordinatensystem:

$$\vec{n}(t, \delta) = \vec{s}(t, \delta) + \vec{p}(t, \delta)$$

$$\vec{n}\left(t,\delta\right) = \begin{bmatrix} l - d + x \\ \sqrt{l_s^2 - \left(l - d + x - p_x\left(t,\delta\right)\right)^2} + p_y\left(t,\delta\right) \end{bmatrix} \qquad (0\text{-}34)$$

mit e, l, x, $l_s$ = const.

Der nicht erneut dargestellte Ortsvektor e sowie der Geschwindigkeitsvektor v des Kolbens und der Winkelvektor $\varphi$, lassen sich wie bei der Ausführung 1 ermitteln, siehe dort.

Das Prinzip des Beispiels 14.7 beruht darauf, dass der Gleitstift C die Eigenschaften bzw. Zwangsbedingungen der ersten Ausführung anteilig mit der zweiten kombiniert.

Um dies zu bewirken, existiert ein Hilfspleuel $B_1$C bzw. b, welches die Gleitbewegung des Gleitstifts C erzwingt. Der Gleitstift C gleitet sowohl in einer Nut in der Wand, die bezüglich der Nulllage unter einem Höhenwinkel $\delta$ längs in Richtung AC verläuft, als auch auf der Längsachse des Pleuels, zum Beispiel in einer Nut im Pleuel. Der Winkel $\delta$ sei einstellbar, aber als zeitlich konstant anzusehen. Er stellt die Variable für die Hubverstellung dar. Das Bezugskoordinatensystem hat seinen Ursprung in der Symmetrieachse der Motorwelle, dem Punkt A. Der Vektor e stellt die Verbindung zwischen dem Punkt A und dem Mittelpunkt des unteren Pleuelauges B des Hauptpleuels l dar. Sein Betrag sei e und zeitlich konstant. Der Vektor $e_1$ stellt die Verbindung zwischen dem Punkt A und dem Mittelpunkt des unteren Pleuelauges $B_1$ des Hilfspleuels b dar. Sein zeitlich konstanter Betrag sei $e_1$, ein prozentualer und konstruktiv festzulegender Anteil von e. Der Vektor b stellt schematisch ein Hilfspleuel dar, sein Betrag sei b und errechnet sich zu b = e + $l_1$ - $e_1$. Der Vektor d stellt die Koordinaten des Gleitstifts C dar, sein Betrag sei d und zeitlich veränderlich. Er schließt den oben genannten Höhenwinkel $\delta$ mit der Abszisse ein. Der Vektor l stellt die schematische Reduktion des Hauptpleuels dar und verbindet den Punkt B über den Gleitstift C hinweg mit dem Punkt D. Der Betrag von l sei l. Der Vektor p zeigt auf die Koordinaten des oberen Hauptpleuelauges D. Der Vektor s stellt die Längsachse der Schubstange DE dar, welche das obere Hauptpleuelauge D mit dem Kolben E verbindet, sein Betrag sei $l_s$. Der Kolben E, welcher unter anderem die Nadel aufnehmen soll, besitzt eine feste x-Koordinate, lediglich die y-Koordinate ist zeitvariant. Der Vektor n zeigt dabei auf die Koordinaten des Kolbens E bezüglich des Koordinatensystems.

Wegen des größeren baulichen Aufwands - es sind mehr bewegliche Teile und damit Fehlerquellen im System - kann die Realisierbarkeit im Einzelfall schwieriger als bei den Beispielen 14.1 bis 14.4 sein.

Die Auswirkung einer zeitlich konstanten, nicht verschwindenden Phasenbeziehung $\lambda$ zwischen den Vektoren e und $e_1$ wurde hier nicht untersucht, könnte sich jedoch als vorteilhaft erweisen. Ebenso eine andere Länge b für das Hilfspleuel. Der erstgenannte Ansatz ist in Beispiel 14.7 veranschaulicht. Hierbei sind für $\lambda$ sowohl positive als auch negative Werte denkbar.

Der in seinem Betrag zeitveränderliche Vektor d, welcher vom Koordinatenursprung A zum Gleitstift C zeigt, ergibt sich in Anlehnung an die Ausführung 1 unter Anwendung des Sinussatzes für beliebige Dreiecke, der Beziehung für die Innenwinkelsumme sowie der Einbeziehung einer nicht verschwindenden Phasenbeziehung $\lambda$ zu:

$$\vec{d}\left(t,\delta\right) = \underbrace{\sqrt{e_1^2 + b^2 - 2\cdot e_1 \cdot b\cdot \cos\left(\gamma\left(t\right)\right)}}_{=\left|\vec{d}\left(t,\delta\right)\right|;\ \text{folgt aus dem Kosinussatz für beliebige Dreiecke}} \cdot \begin{bmatrix} \cos\left(\delta\right) \\ \sin\left(\delta\right) \end{bmatrix} \qquad (0\text{-}35)$$

mit b = e - $e_1$ + $l_1$
mit $e_1$, b, $\delta$ = const.

$$\text{mit } \gamma(t) = \pi - \left[ \arcsin\left( \underbrace{\frac{e}{l_1} \cdot \sin\big(\varphi(t) - \lambda - \delta\big)}_{= \sin(\alpha),\ \text{Sinussatz}} \right) \atop \underbrace{\phantom{\arcsin\left(\frac{e}{l_1}\sin\right)}}_{= \alpha,\ \text{Innenwinkel 1}} + \underbrace{\big(\varphi(t) - \lambda - \delta\big)}_{\text{Innenwinkel 2}} \right]$$

$$\underbrace{\phantom{XXXXXXXXXXXXXXXXXXX}}_{\text{folgt aus Innenwinkelsumme im beliebigen Dreieck}}$$

Somit lässt sich der Vektor l ähnlich zur Ausführung 2 unter Verwendung seines Betrags l errechnen:

$$\vec{l}(t,\delta) = l \cdot \underbrace{\frac{\vec{d}(t,\delta) - \vec{e}(t)}{\left| \vec{d}(t,\delta) - \vec{e}(t) \right|}}_{\substack{= \text{normierter Vektor} \\ \text{in Pleuelrichtung}}} \quad \text{mit } l = \text{const.} \qquad (0\text{-}36)$$

Die nicht erneut dargestellten Ortsvektoren e, p, s und n sowie der Geschwindigkeitsvektor v des Kolbens und der Winkelvektor φ.

Im Unterschied zu Ausführung 14.2 gleitet bei dem Beispiel 14.8 eine um den Betrag q parallel zur Pleuellängsachse versetzte Hilfsachse in einer drehbar gelagerten, wandfesten Hülse. Damit gehen beide Ausführungen bei q = 0 ineinander über. Die schematischen Vektorbeziehungen sind in Beispiel 14.8 dargestellt.

Bei dieser Ausführung war die Modellbildung ein wenig aufwendiger. Stellt man sich jedoch den Pleuel reduziert auf ein Rechteck vor, welches auf einer Gleitrolle C mit infinitesimalem Durchmesser aufliegt, so vereinfacht sich die Vektorerstellung.

Es wird angenommen, dass die Vektoren *e, d* und damit y sowie deren Beträge jederzeit bekannt seien, vgl. hierzu Beispiel 14.8. Der Betrag q des Vektors q sei ebenfalls bekannt, denn q stellt den konstruktiv gewünschten Parallelversatz dar. Die Vektoren y, q und z spannen ein rechtwinkliges Dreieck auf. Somit kann die Arcussinusfunktion verwendet werden, um den Winkel ζ zu berechnen. Dieser tritt wegen der Parallelität von z und 1 als Wechselwinkel ebenfalls zwischen y und l auf. Damit muss, um den Vektors l zu erhalten, abschließend der Vektor y auf die Länge l normiert, um ζ im positiven Sinn gedreht und mit dem Betrag l skaliert werden. Der so erhaltene Vektor l stellt wie bei den Ausführungn zuvor die Verbindung BD her. Die übrigen Verhältnisse und Vektoren berechnet man wie bereits in der Ausführung 14.1.

Für den Vektor d gilt, dass bei δ = 0 der Vektor mit der positiven Abszisse einen Winkel $\delta_0$ einschließt. Seine Koordinaten sind in diesem Fall (d, -q), wobei q aus der konstruktiven Vorgabe und d aus der Berechnung $d = e + l_1$ erhalten werden. Für einen Höhenwinkel δ, der für eine superponierte Hubverstellung anzupassen ist auf eine günstige Arbeitslage, muss der so erhaltene Vektor $d_0(\delta_0)$ mithilfe der 2D-Drehmatrix in der Ebene gedreht werden, man erhält den Vektor $d(\delta_0+\delta)$. Die Koordinaten nach einer Drehung seien (d*, -q*), was in Fig. 14a dargestellt ist.

$$\vec{d}(\delta) = \underbrace{\begin{bmatrix} \cos(\delta) & -\sin(\delta) \\ \sin(\delta) & \cos(\delta) \end{bmatrix}}_{\substack{\text{2D-Drehmatrix} \\ \text{Drehung um } \square\ \delta}} \cdot \underbrace{\begin{bmatrix} d \\ -q \end{bmatrix}}_{\substack{\text{Vektor} \\ d_0}} \qquad (0\text{-}37)$$

mit d, q = const.

Man erhält den Vektor y zu:

$$\vec{y}(t, \delta) = \vec{d}(\delta) - \vec{e}(t) \qquad (0\text{-}38)$$

Damit lässt sich der Vektor l ermitteln:

$$\vec{l}(t, \delta) = 1 \cdot \underbrace{\begin{bmatrix} \cos(\zeta(t, \delta)) & -\sin(\zeta(t, \delta)) \\ \sin(\zeta(t, \delta)) & \cos(\zeta(t, \delta)) \end{bmatrix}}_{\substack{\text{2D-Drehmatrix} \\ \text{Drehung um } \square\ \zeta(t, \delta)}} \cdot \underbrace{\frac{\vec{y}(t, \delta)}{|\vec{y}(t, \delta)|}}_{\substack{= \text{normierter} \\ \text{y-Vektor}}} \qquad (0\text{-}39)$$

mit l = const.

mit

$$\zeta(t, \delta) = \arcsin\left(\frac{q}{|\vec{y}(t, \delta)|}\right)$$

Die nicht erneut dargestellten Ortsvektoren e, p, s und n sowie der Geschwindigkeitsvektor v des Kolbens und der Winkelvektor $\varphi$, lassen sich wie bei Ausführung 1 ermitteln, siehe dort.

Zudem kann die Forderung nach einem konstanten Parallelversatz q aufgegeben werden. Dieser wäre dann eine Funktion $q(|z(t)|)$. Denkbar ist eine zur Pleuellängsachse schräg-lineare oder auch eine frei geformte Gleitnut im Pleuel. Die analytische Lösung ist zum Beispiel mit folgendem Vorgehen möglich: Der Betrag von y sei für jedes t bekannt bzw. berechenbar. Man nehme den Betrag von z und damit $q(z)$ zunächst als bekannt an, dann lässt sich über den Satz des Pythagoras $y^2 = q^2(|z|) + |z|^2$ formulieren. Für die Ermittlung von z müssen folgende Bedingungen Beachtung finden, da eventuell mehrere Lösungen erhalten werden, zum Beispiel als Nullstellen eines Polynoms.

$$\underbrace{\vec{y}(t, \delta) \leq q(z) + z(t, \delta)}_{\text{Dreiecksungleichung}} \quad \text{und} \quad \underbrace{z(t, \delta) \geq 0}_{\substack{\text{keine} \\ \text{negativen Werte}}} \quad \text{und} \quad \underbrace{z(t, \delta) \in \Re}_{\substack{\text{keine} \\ \text{komplexen Werte}}} \qquad (0\text{-}40)$$

Falls diese Beziehung nach $|z|$ aufgelöst oder numerisch gelöst werden kann, ist die Ermittlung des Winkels $\zeta$ über die arccos-Funktion möglich. Das weitere Vorgehen entspricht dem in der Gleichung (0-39).

Eine Erweiterung dessen um das Konzept aus der Ausführung 7 ist zusätzlich denkbar, wurde hier jedoch nicht untersucht. Dabei würde der Gleitstift C mittels eines Hilfspleuels, welches durch eine Hilfskurbel $e_1$ mit fester Phasenbeziehung $\lambda$ zur Hauptkurbel e getrieben wird, in einer Nut in der Gehäusewand und / oder in der Pleuelnut geführt werden. Die jeweilige Nut könnte statt einer geraden eine freie Form aufweisen.

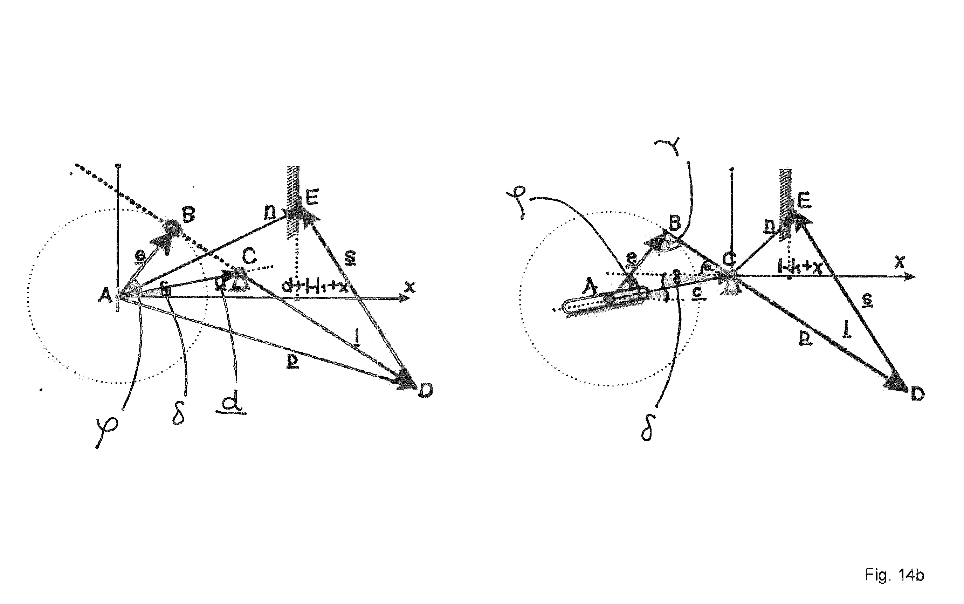

Das Beispiel 14.9 (vgl. Fig. 14b) kombiniert Eigenschaften aus der Ausführung 14.2 und der Ausführung 14.5 (vgl. Fig. 14a). Dabei dreht die Antriebskurbel um den Punkt A und greift wie bei der Ausführung 14.2 am Punkt B an, der Umlenkstift C wird von einer weiteren, um den Punkt A drehenden Kurbel, ähnlich der Antriebskurbel in Ausführung 14.5, bewegt. Dabei gleitet der Stift C auf der Pleuellängsachse oder in einer Pleuelnut.

Hier wird angenommen, dass beide Antriebskurbeln über eine Verzahnung miteinander verbunden sind und eine beliebige, aber zeitlich konstante Phase $\lambda$ (= Drehversatz) vorliegt. Das Übersetzungsverhältnis n der Zahnradpaarung $z_1$ und $z_2$ muss nicht zwingend Eins betragen, dies vereinfacht jedoch die Mechanik und die Konstruktion und wird hier angenommen. Bei einer Unter- oder Übersetzung der Antriebsdrehzahl erhält man eine mit entsprechender Periodik wechselnde Dynamik des Kolbens, was vorteilhaft bezüglich der Schwingungsdynamik der Haut sein kann. Zusätzlich ist für den Fall $n \neq 1$ der Kurbelradius unbedingt kleiner als der Kopfkreisdurchmesser der Zahnräder zu wählen, um eine Kollision der Punkte B und C zu vermeiden, wie man in der Darstellung erkennt.

Für die Kurbeldrehwinkel $\varphi$ und $\delta$ und die Zähnezahl $z_1$ und $z_2$ gilt ohne Ausschluss der Allgemeinheit:

$$\delta(t) = \frac{z_1}{z_2} \cdot \varphi(t) \qquad\qquad (0\text{-}41)$$

Der hier zeitlich veränderliche Gleitstiftvektor d, welcher vom Koordinatenursprung A zum Umlenkstift C zeigt, ergibt sich als Summe aus dem Umlenkstift-Kurbelvektor r und dem konstanten Abstandsvektor der Kurbelachse a unter Einbeziehung der Phase $\lambda$ zu:

$$\vec{d}(t) = \begin{Bmatrix} a \\ 0 \end{Bmatrix} + \cdot \begin{Bmatrix} \cos\left[\delta(t)+\lambda\right] \\ \sin\left[\delta(t)+\lambda\right] \end{Bmatrix} \; a \; , \quad r \; , \quad \lambda = \text{const.} \qquad (0\text{-}42)$$

Somit lässt sich der Vektor l unter Verwendung seines Betrags l errechnen:

$$\vec{l}(t) = l \cdot \underbrace{\frac{\vec{d}(t) - \vec{e}(t)}{\left|\vec{d}(t) - \vec{e}(t)\right|}}_{\substack{= \text{ normierter Vektor} \\ \text{in Pleuelrichung}}} \quad \text{mit } l = \text{const.} \qquad (0\text{-}43)$$

Es ergibt sich der Ortsvektor p des oberen Pleuelauges D im Koordinatensystem:

$$\vec{p}(t) = \vec{l}(t) + \vec{e}(t) \qquad\qquad (0\text{-}44)$$

Der Lagevektor s der Schubstange bildet mit einer Vertikalen durch den Punkt D und mit der Abszisse durch den Punkt E ein rechtwinkliges Dreieck und kann deshalb mit dem Satz des Pythagoras unter Verwendung der bekannten Länge $l_s$ ermittelt werden, wobei im Folgenden $p_x(t)$ und $p_y(t)$ die zeitveränderlichen Komponenten des Pleuelvektors p darstellen:

$$\vec{s}(t) = \begin{bmatrix} \sqrt{l_s^2 - p_y(t)^2} \\ - p_y(t) \end{bmatrix} \quad \text{mit } l_s = \text{const.} \qquad (0\text{-}45)$$

Als Summe der Vektoren p und s ergibt sich schließlich der Vektor n des Kolbens E im Koordinatensystem:

$$\vec{n}(t) = \vec{s}(t) + \vec{p}(t)$$

$$\vec{n}(t) = \begin{bmatrix} \sqrt{l_s^2 - p_y(t)^2} + p_x(t) \\ 0 \end{bmatrix} \qquad (0\text{-}46)$$

Der nicht erneut dargestellte Ortsvektor e sowie der Geschwindigkeitsvektor v des Kolbens und der Winkelvektor φ, lassen sich wie bei der Ausführung 1 ermitteln, siehe dort.

Das Beispiel 14.9 aus Fig. 14b bietet eine gute und konstruktiv einfach zu realisierende Möglichkeit, ein Stechgerät mit sich periodisch und ständig ändernder Nadelgeschwindigkeit aufzubauen. Weitere Vorteile können sich ergeben, wenn die Drehachse der Umlenkstift-Kurbel um den Punkt A von der Abszisse weg verlagert wird. Dies kann ebenfalls konstruktiv einfach realisiert werden. Das komplexe Zusammenspiel der möglichen Änderungen wurde bisher nicht untersucht.

Nachfolgend werden weitere Aspekte der Hubeinstellung mit kompensiertem und einstellbarem Nadelherausstand bei hoher Stechgeschwindigkeit näher erläutert. Es wurde das Beispiel 14.5 ausgewählt, da hier die Hubeinstellung mit Kompensation gut umsetzbar ist.

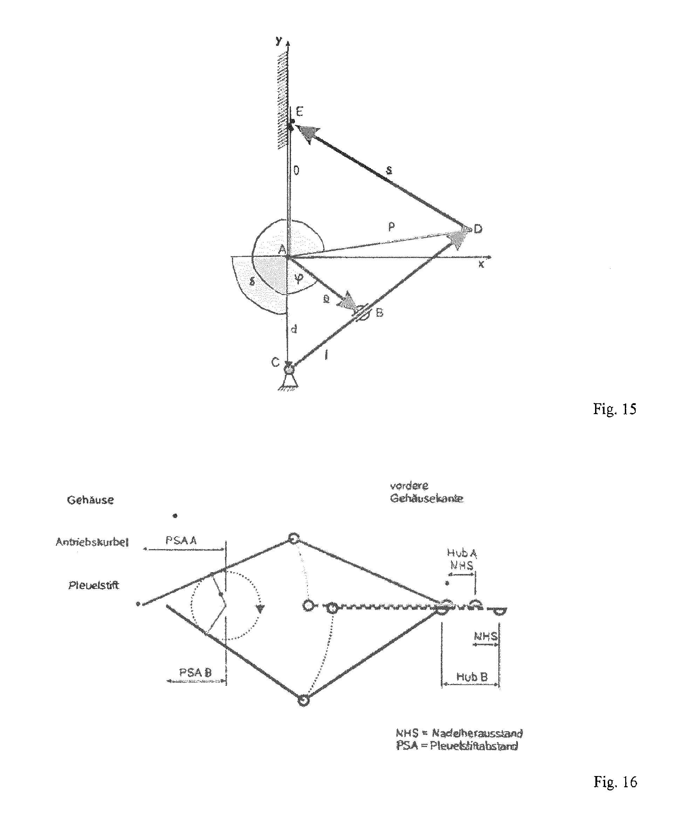

Bei dem Beispiel werden im Vergleich zum Beispiel 14.2 die Rolle des Gleitstifts C und der Kurbel B vertauscht. Zusätzlich wurde zugunsten einer alternierenden Stechfrequenz (siehe weiter unten) ein Höhenwinkel δ = 90° gewählt und auf den seitlichen Versatz l - d + x (Variante 5) verzichtet, was die schematische Darstellung in Fig. 15 zeigt. Dies bewirkt, dass bei jeder 360°-Drehung der Kurbel AB der Pleuel CD eine Bewegung ähnlich einem Pendel ausführt und dabei das obere Pleuelauge D eine um die Mittellage symmetrische Kreisbogenbahn zweifach überstreicht. Dabei unterscheidet sich bei konstanter Motordrehzahl die maximale Geschwindigkeit der einen Pendelhalbperiode erheblich von der der anderen. Somit werden bei jeder Motordrehung zwei Stechbewegungen mit unterschiedlicher Geschwindigkeit und, daraus resultierend, mit alternierend wechselnder Stechfrequenz erzeugt. Dies verhindert beim Stechen, dass die Haut als schwingfähiges System zu einer gleichlaufenden Schwingung angeregt wird.

Die Hubeinstellung wird bei dieser Gestaltungsvariante durch eine Veränderung des Abstands zwischen dem Pleueldrehpunkt C und der Motorwelle A realisiert, wodurch sich der von den durch C laufenden Tangenten an den Kurbelkreis eingeschlossene Winkel und damit die Lage des unteren (hinteren) Totpunkts des Nadelkolbens E ändern.

Anhand der schematischen Darstellung in Fig. 16 wird die NHS-kompensierte Hubeinstellung erläutert, wobei die Verhältnisse in Fig. 15 um 90° rechtsläufig gedreht wurden. Dargestellt sind das Pleuel und die Schubstange in den Totpunkten der Stechbewegung für zwei Hubeinstellungen "Fall A" (obere Bildhälfte) und "Fall B" (untere Bildhälfte). Im "Fall A" ist der Pleuelstift-Abstand (PSA) zur Motorwelle größer als im "Fall B", wodurch der bei einer vollständigen Pleuelschwingung durch die Tangenten aufgespannte Winkel und der Hub der Nadelbewegung kleiner sind und umgekehrt. Vorteilhaft bei der dargestellten Gestaltungsvariante für die Hubeinstellung mit dabei in festem Abstand mitbewegter Gehäusespitze ist, dass der Abstand zwischen dem Pleueldrehpunkt und dem oberen (vorderen) Totpunkt unabhängig vom eingestellten Hub bleibt, sodass der vom Anwender gewählte Nadelherausstand (NHS) und damit die theoretisch mögliche Stechtiefe unverändert bleiben und keiner weiteren Ausgleichsmechanik bedarf.

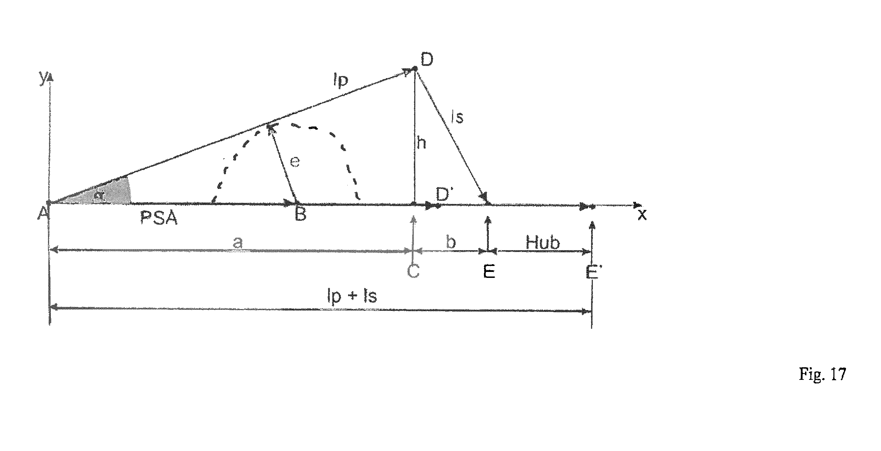

Für die analytische Ermittlung des Hubs in Abhängigkeit vom Pleuelstiftabstand (PSA) wird auf Fig. 17. Bezug genommen. Diese zeigt die Verhältnisse für den vorderen E' und hinteren E Totpunkt der Nadel. Die Kurbel e rotiert um den Punkt B, der Pleuel dreht um den Stellstift A.

Dabei kann für das System im hinteren Totpunkt E über den Strecken AB und AC ein rechtwinkliges Dreieck mit einem gemeinsamen Innenwinkel α aufgespannt werden, sodass gilt:

$$\sin(\alpha) = \frac{e}{PSA} = \frac{h}{l_p} \qquad (0\text{-}47)$$

Daraus ergibt sich h zu

$$h = \frac{l_P \cdot e}{PSA} \qquad (0\text{-}48)$$

Des Weiteren erhält man für die Summe der Strecken a (= AC) und b (=CE) unter Anwendung des Satzes des Pythagoras folgende Beziehung:

$$a + b = \underbrace{\sqrt{l_P{}^2 - h^2}}_{=\,a} + \underbrace{\sqrt{l_S{}^2 - h^2}}_{=\,b} \qquad (0\text{-}49)$$

Dabei ist $l_P$ die Länge des Pleuels, h die Höhe und $l_S$ die Länge der Schubstange. Wird (0-48) in (0-49) eingesetzt, so kann für die konstruktiv festgelegten Größen e, $l_P$ und $l_S$ eine Abhängigkeit des Hubs von der veränderlichen Größe PSA angegeben werden.

$$Hub = f\left(PSA\right) = l_P + l_s - \left(a + b\right) \qquad (0\text{-}50)$$

$$Hub = f\left(PSA\right) = l_P + l_s - \underbrace{\sqrt{l_P{}^2 - \left(\frac{l_P \cdot e}{PSA}\right)^2}}_{a} - \underbrace{\sqrt{l_S{}^2 - \left(\frac{l_P \cdot e}{PSA}\right)^2}}_{b} \qquad (0\text{-}51)$$

In einer Ausführung wurden zum Beispiel ein Kurbelkreisradius e = 2 mm, eine Pleuellänge $l_P$ = 15 mm und für die Schubstange eine Länge $l_S$ = 11 mm gewählt.

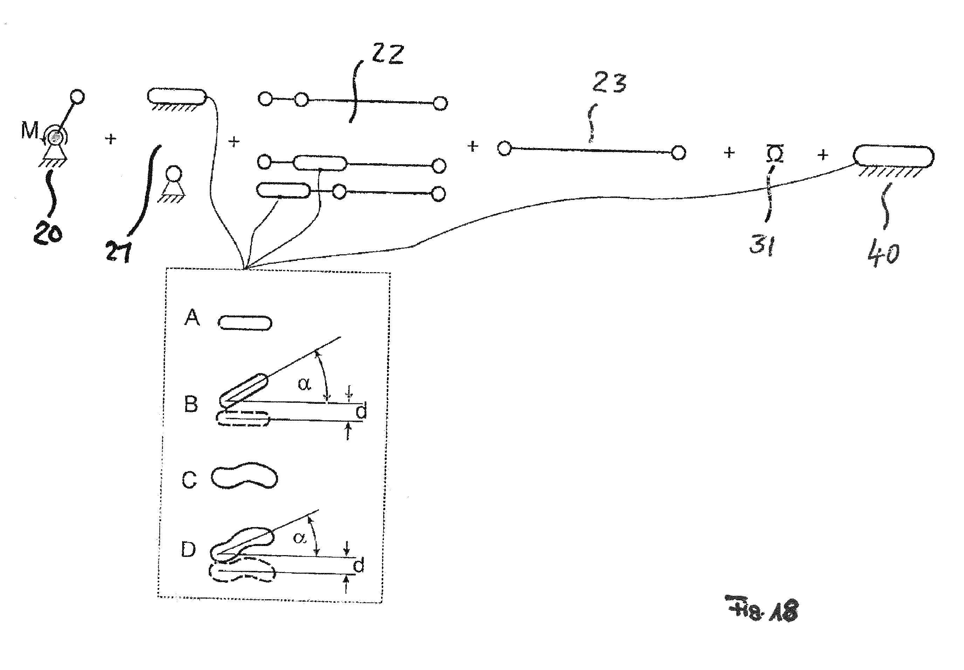

Fig. 18 zeigt schematisch und beispielhaft eine zusammenfassende Darstellung für unterschiedliche Ausgestaltungen des Wandlungsmechanismus zum Wandeln der rotierenden Antriebsbewegung in die axial ausgerichtete Antriebsbewegung, wobei die Funktionselemente Kurbeleinrichtung 20, Gehäuselagerung 27, proximales Pleuelelement 22, distales Pleuelelement 23 sowie Kolbenelement 31 schematisch gezeigt sind. In den gezeigten Beispielen kann eine weitere Gehäuselagerung 40 vorgesehen sein. Die für die jeweiligen Funktionselemente gezeigten Ausführungsformen können beliebig miteinander kombiniert werden. So sind für das proximale Pleuelelement 22 drei unterschiedliche Ausgestaltungsformen gezeigt, von denen zwei Ausführungen ein Verschiebe- oder Gleitlager aufweisen.

Die in der vorstehenden Beschreibung, den Ansprüchen sowie der Zeichnung offenbarten Merkmale können sowohl einzeln als auch in beliebiger Kombination für die Verwirklichung der verschiedenen Ausführungen von Bedeutung sein.

**Patentansprüche**

1. Handgerät zum wiederholten lokalen Aufstechen einer menschlichen oder tierischen Haut, mit:

- einem Gehäuse (2), an dem ein Handgriff (3) gebildet ist,
- einer Antriebseinrichtung, die in dem Gehäuse (2) angeordnet ist und mit der über eine Antriebswelle eine rotierende Antriebsbewegung bereitgestellt wird,
- einem an die Antriebswelle koppelnden Wandlungsmechanismus, der in dem Gehäuse (2) angeordnet und eingerichtet ist, die rotierende Antriebsbewegung in eine axial ausgerichtete Antriebsbewegung zu wandeln, und
- einer Stecheinrichtung, die in dem Gehäuse (2) angeordnet ist und eine oder mehrere Stechnadeln aufweist, welche an einem Nadelschaft aufgenommen sind, der mit der einen oder den mehreren Nadeln entlang einer Bewegungsbahn wiederholt vor und zurück bewegbar ist und mit dem Wandlungsmechanismus in Verbindung steht,

**dadurch gekennzeichnet, dass** der Wandlungsmechanismus eine Kurbeleinrichtung (20) aufweist, die an die Antriebswelle koppelt, und eine Reihenanordnung (21) von mehreren gelenkig miteinander gekoppelten Pleuelelementen aufweist, wobei bei der Reihenanordnung (21) ein proximales Pleuelelement (22) mit der Kurbeleinrichtung (20) und ein distales Pleuelelement (23) mit dem Nadelschaft in Verbindung steht, derart, dass im Betrieb die axiale ausgerichtete Antriebsbewegung auf den Nadelschaft eingeleitet wird.

2. Handgerät nach Anspruch 1, **dadurch gekennzeichnet, dass** der Wandlungsmechanismus eine Gehäuselagerung (27) aufweist, mit der das proximale Pleuelelement (22) am Gehäuse (2) gelagert ist.

3. Handgerät nach Anspruch 2, **dadurch gekennzeichnet, dass** das proximale Pleuelelement (22) an der Gehäuselagerung (27) um eine Schwenkachse schwenkbar gelagert ist.

4. Handgerät nach Anspruch 2 oder 3, **dadurch gekennzeichnet, dass** das proximale Pleuelelement (22) mittels der Gehäuselagerung (27) entlang einer Führung, die am Gehäuse (2) oder an dem proximalen Pleuelelement (22) gebildet ist, verschiebbar gelagert ist.

5. Handgerät nach mindestens einem der Ansprüche 2 bis 4, **dadurch gekennzeichnet, dass** die Gehäuselagerung (27) auf einer von dem Nadelschaft abgewandten Seite der Kurbeleinrichtung (20) am Gehäuse gebildet ist.

6. Handgerät nach mindestens einem der vorangehenden Ansprüche, **dadurch gekennzeichnet, dass** ein Koppelelement (26) der Kurbeleinrichtung (20) in einer Pleuelelementführung (25) an dem proximalen Pleuelelement (22) verschiebbar lagert.

7. Handgerät nach mindestens einem der vorangehenden Ansprüche, **dadurch gekennzeichnet, dass** zumindest ein Pleuelelement der Reihenanordnung (21) einen axialen Längenausgleich zulassend ausgeführt ist.

8. Handgerät nach mindestens einem der vorangehenden Ansprüche, **dadurch gekennzeichnet, dass** die Reihenanordnung (21) aus dem proximalen Pleuelelement (22) und dem distalen Pleuelelement (23) besteht.

9. Handgerät nach mindestens einem der vorangehenden Ansprüche, dadurch **gekenn - zeichnet,** dass eine Hubeinstellvorrichtung (34, 35) vorgesehen ist, mit der ein Hub der axial ausgerichteten Antriebsbewegung einstellbar ist.

10. Handgerät nach Anspruch 2 und 9, **dadurch gekennzeichnet, dass** mit Hilfe der Hubeinstellvorrichtung (34, 35) die Gehäuselagerung (27) verstellbar ist.

11. Handgerät nach Anspruch 9 oder 10, **dadurch gekennzeichnet, dass** die Hubeinstellvorrichtung (34, 35) eine am Gehäuse (2) außenliegend angeordnete Einstelleinrichtung (35) aufweist.

12. Handgerät nach mindestens einem der vorangehenden Ansprüche, **dadurch gekennzeichnet, dass** die axial ausgerichtete Antriebsbewegung quer zur axialen Richtung der Antriebswelle verläuft.

13. Handgerät nach mindestens einem der vorangehenden Ansprüche, dadurch **gekenn - zeichnet,** dass das Gehäuse (2) mit mehreren Gehäusemodulen gebildet ist, wobei in einem Antriebsmodul (5) die Antriebseinrichtung und in einem Stechmodul (6) die Stecheinrichtung angeordnet ist.

14. Handgerät nach Anspruch 13, **dadurch gekennzeichnet, dass** der Wandlungsmechanismus teilweise in dem Antriebsmodul (5) und teilweise in dem Stechmodul (6) gebildet ist.

15. Antriebsmodul für ein Handgerät zum wiederholten lokalen Aufstechen einer menschlichen oder tierischen Haut, mit:

- einem Modulgehäuse, an dem ein Handgriff gebildet ist,
- einer Antriebseinrichtung, die in dem Modulgehäuse angeordnet ist und mit der über eine Antriebswelle eine rotierende Antriebsbewegung bereitgestellt wird,
- einem an die Antriebswelle koppelnden Wandlungsmechanismus, der in dem Modulgehäuse angeordnet und eingerichtet ist, die rotierende Antriebsbewegung in eine axial ausgerichtete Antriebsbewegung zu wandeln, und
- einer Kopplungseinrichtung, die an und / oder in dem Modulgehäuse gebildet und eingerichtet ist, an ein Nadelmodul lösbar zu koppeln,

**dadurch gekennzeichnet, dass** der Wandlungsmechanismus eine Kurbeleinrichtung (20), die an die Antriebswelle koppelt, und eine Reihenanordnung (21) von mehreren gelenkig miteinander gekoppelten Pleuelelementen aufweist, wobei die Reihenanordnung (21) mit einem proximalen Pleuelelement (22), welches mit der Kurbeleinrichtung (20) in Verbindung steht, und einem distalen Pleuelelement (23), welches an einen Nadelschaft einer Stecheinrichtung koppelbar ist, gebildet ist eine Nadelschaft, derart, dass im Betrieb am distalen Pleuelelement (23) die axial ausgerichtete Antriebsbewegung bereitgestellt ist.

**Geänderte Patentansprüche gemäss Regel 137(2) EPÜ.**

1. Handgerät zum wiederholten lokalen Aufstechen einer menschlichen oder tierischen Haut, mit:

   - einem Gehäuse (2), an dem ein Handgriff (3) gebildet ist,
   - einer Antriebseinrichtung, die in dem Gehäuse (2) angeordnet ist und mit der über eine Antriebswelle eine rotierende Antriebsbewegung bereitgestellt wird,
   - einem an die Antriebswelle koppelnden Wandlungsmechanismus, der
   - in dem Gehäuse (2) angeordnet und eingerichtet ist, die rotierende Antriebsbewegung in eine axial ausgerichtete Antriebsbewegung zu wandeln, und
   - eine Kurbeleinrichtung (20) aufweist, die an die Antriebswelle koppelt, und
   - einer Stecheinrichtung, die in dem Gehäuse (2) angeordnet ist und eine oder mehrere Stechnadeln aufweist, welche an einem Nadelschaft aufgenommen sind, der mit der einen oder den mehreren Nadeln entlang einer Bewegungsbahn wiederholt vor und zurück bewegbar ist und mit dem Wandlungsmechanismus in Verbindung steht,

   **dadurch gekennzeichnet, dass** der Wandlungsmechanismus

   - eine Reihenanordnung (21) von mehreren gelenkig miteinander gekoppelten Pleuelelementen aufweist, bei der ein proximales Pleuelelement (22) mit der Kurbeleinrichtung (20) und ein distales Pleuelelement (23) mit dem Nadelschaft in Verbindung steht, derart, dass im Betrieb die axial ausgerichtete Antriebsbewegung auf den Nadelschaft eingeleitet wird, und
   - eine Gehäuselagerung (27) aufweist, mit der das proximale Pleuelelement (22) am Gehäuse (2) gelagert ist.

2. Handgerät nach Anspruch 1, **dadurch gekennzeichnet, dass** das proximale Pleuelelement (22) an der Gehäuselagerung (27) um eine Schwenkachse schwenkbar gelagert ist.

3. Handgerät nach Anspruch 1 oder 3, **dadurch gekennzeichnet, dass** das proximale Pleuelelement (22) mittels der Gehäuselagerung (27) entlang einer Führung, die am Gehäuse (2) oder an dem proximalen Pleuelelement (22) gebildet ist, verschiebbar gelagert ist.

4. Handgerät nach mindestens einem der Ansprüche 1 bis 3, **dadurch gekennzeichnet, dass** die Gehäuselagerung (27) auf einer von dem Nadelschaft abgewandten Seite der Kurbeleinrichtung (20) am Gehäuse gebildet ist.

5. Handgerät nach mindestens einem der vorangehenden Ansprüche, **dadurch gekennzeichnet, dass** ein Koppelelement (26) der Kurbeleinrichtung (20) in einer Pleuelelementführung (25) an dem proximalen Pleuelelement (22) verschiebbar lagert.

6. Handgerät nach mindestens einem der vorangehenden Ansprüche, **dadurch gekennzeichnet, dass** zumindest ein Pleuelelement der Reihenanordnung (21) einen axialen Längenausgleich zulassend ausgeführt ist.

7. Handgerät nach mindestens einem der vorangehenden Ansprüche, **dadurch gekennzeichnet, dass** die Reihenanordnung (21) aus dem proximalen Pleuelelement (22) und dem distalen Pleuelelement (23) besteht.

8. Handgerät nach mindestens einem der vorangehenden Ansprüche, **dadurch gekennzeichnet, dass** eine Hubeinstellvorrichtung (34, 35) vorgesehen ist, mit der ein Hub der axial ausgerichteten Antriebsbewegung einstellbar ist.

9. Handgerät nach Anspruch 8, **dadurch gekennzeichnet, dass** mit Hilfe der Hubeinstellvorrichtung (34, 35) die Gehäuselagerung (27) verstellbar ist.

10. Handgerät nach Anspruch 8 oder 9, **dadurch gekennzeichnet, dass** die Hubeinstellvorrichtung (34, 35) eine am Gehäuse (2) außenliegend angeordnete Einstelleinrichtung (35) aufweist.

11. Handgerät nach mindestens einem der vorangehenden Ansprüche, **dadurch gekennzeichnet, dass** die axial ausgerichtete Antriebsbewegung quer zur axialen Richtung der Antriebswelle verläuft.

12. Handgerät nach mindestens einem der vorangehenden Ansprüche, **dadurch gekennzeichnet, dass** das Gehäuse (2) mit mehreren Gehäusemodulen gebildet ist, wobei in einem Antriebsmodul (5) die Antriebseinrichtung und in einem Stechmodul (6) die Stecheinrichtung angeordnet ist.

13. Handgerät nach Anspruch 12, **dadurch gekennzeichnet, dass** der Wandlungsmechanismus teilweise in dem Antriebsmodul (5) und teilweise in dem Stechmodul (6) gebildet ist.

14. Antriebsmodul für ein Handgerät zum wiederholten lokalen Aufstechen einer menschlichen oder tierischen Haut, mit:

   - einem Modulgehäuse, an dem ein Handgriff gebildet ist,
   - einer Antriebseinrichtung, die in dem Modulgehäuse angeordnet ist und mit der über eine Antriebswelle eine rotierende Antriebsbewegung bereitgestellt wird,
   - einem an die Antriebswelle koppelnden Wandlungsmechanismus, der
   - in dem Modulgehäuse angeordnet und eingerichtet ist, die rotierende Antriebsbewegung in eine axial ausgerichtete Antriebsbewegung zu wandeln, und
   - eine Kurbeleinrichtung (20) aufweist, die an die Antriebswelle koppelt, und'
   - einer Kopplungseinrichtung, die an und / oder in dem Modulgehäuse gebildet und eingerichtet ist, an ein Nadelmodul lösbar zu koppeln,

   **dadurch gekennzeichnet, dass** der Wandlungsmechanismus

   - eine Reihenanordnung (21) von mehreren gelenkig miteinander gekoppelten Pleuelelementen aufweist, wobei die Reihenanordnung (21) mit einem proximalen Pleuelelement (22), welches mit der Kurbeleinrichtung (20) in Verbindung steht, und einem distalen Pleuelelement (23), welches an einen Nadelschaft einer Stecheinrichtung koppelbar ist, gebildet ist eine Nadelschaft, derart, dass im Betrieb am distalen Pleuelelement (23) die axial ausgerichtete Antriebsbewegung bereitgestellt ist, und
   - eine Gehäuselagerung (27) aufweist, mit der das proximale Pleuelelement (22) am Gehäuse (2) gelagert ist.

Fig. 1

Fig. 2

Fig. 3

Fig. 4

EP 2 954 925 A1

Fig. 5

29

Fig. 6

Fig. 7

Fig. 8

Fig. 9

Fig. 10

Fig. 11

Fig 12

Fig. 13

Fig. 14a

Fig. 14b

Fig. 14c

Variante 7

Variante 8

Fig. 14d

EP 2 954 925 A1

Fig. 14 e

Fig. 15

NHS = Nadelherausstand
PSA = Pleuelstiftabstand

Fig. 16

Fig. 17

Fig. 8V

Europäisches
Patentamt

European
Patent Office

Office européen
des brevets

**EUROPÄISCHER RECHERCHENBERICHT**

Nummer der Anmeldung

EP 14 17 2119

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 8 522 647 B1 (DIXON ALAN B [US]) 3. September 2013 (2013-09-03) * Abbildungen 1-5 * * Spalte 2, Zeile 54 - Spalte 4, Zeile 9 * ----- | 1-15 | INV. A61M37/00 |
| A | DE 20 2004 016876 U1 (GRUBE ANDREAS [DE]) 21. Juli 2005 (2005-07-21) * Ansprüche 1-6; Abbildung 1 * ----- | 1-15 | |
| A | WO 2014/065726 A1 (INK MACHINES SWEDEN AB [SE]) 1. Mai 2014 (2014-05-01) * Abbildungen 1, 4, 5a, 5b, 6a-6d * ----- | 1-15 | |
| A | DE 43 31 442 A1 (SCHUH STEFAN [DE]) 23. März 1995 (1995-03-23) * Abbildung 3 * ----- | 1-15 | |
| A | GB 1 587 519 A (LANGLEY K) 8. April 1981 (1981-04-08) * Abbildungen 1, 2 * ----- | 1-15 | RECHERCHIERTE SACHGEBIETE (IPC) A61M |

Der vorliegende Recherchenbericht wurde für alle Patentansprüche erstellt

| Recherchenort | Abschlußdatum der Recherche | Prüfer |
|---|---|---|
| Berlin | 17. November 2014 | Przykutta, Andreas |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
..................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 14 17 2119

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

17-11-2014

| Im Recherchenbericht angeführtes Patentdokument | Datum der Veröffentlichung | Mitglied(er) der Patentfamilie | Datum der Veröffentlichung |
|---|---|---|---|
| US 8522647      B1 | 03-09-2013 | KEINE | |
| DE 202004016876 U1 | 21-07-2005 | KEINE | |
| WO 2014065726   A1 | 01-05-2014 | KEINE | |
| DE 4331442      A1 | 23-03-1995 | KEINE | |
| GB 1587519      A | 08-04-1981 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 29919199 U1
- EP 1495782 A1